# EUROPÄISCHE PATENTSCHRIFT

(11) **EP 3 553 159 B1**
(45) Veröffentlichungstag und Bekanntmachung des Hinweises auf die Patenterteilung: **09.06.2021**
(21) Anmeldenummer: 19167875.4
(22) Anmeldetag: 08.04.2019
(51) Int. Cl.: C12M 1/00, C12M 1/12, C12M 1/34

(54) **VORRICHTUNG ZUR HALTERUNG EINER BILDERFASSENDEN EINRICHTUNG AN EINEM BIOREAKTOR, BIOREAKTOR MIT VORRICHTUNG ZUR HALTERUNG EINER BILDERFASSENDEN EINRICHTUNG SOWIE VERFAHREN ZUR VERMEHRUNG ODER KULTIVIERUNG BIOLOGISCHEN MATERIALS**
DEVICE FOR MOUNTING AN IMAGING DEVICE ON A BIOREACTOR, BIOREACTOR WITH DEVICE FOR HOLDING AN IMAGING DEVICE AND METHOD FOR AUGMENTING OR CULTURING BIOLOGICAL MATERIAL
DISPOSITIF DE RETENUE D'UN AGENCEMENT SAISISSANT DES IMAGES SUR UN BIORÉACTEUR, BIORÉACTEUR POURVU DE DISPOSITIF DE RETENUE D'UN AGENCEMENT SAISISSANT DES IMAGES ET PROCÉDÉ DE REPRODUCTION OU DE CULTURE DE LA MATIÈRE BIOLOGIQUE

(30) Priorität: 09.04.2018 DE 102018108323
(43) Veröffentlichungstag der Anmeldung: 16.10.2019
(73) Patentinhaber: SCHOTT AG, 55122 Mainz (DE)
(72) Erfinder: OTT, Christian, 84539 Ampfing (DE); HETTLER, Robert, 84036 Kumhausen (DE)
(74) Vertreter: Blumbach · Zinngrebe Patentanwälte PartG mbB

(56) Entgegenhaltungen:
- WO-A1-2018/014984
- WO-A1-2018/044748
- DE-A1- 3 446 908
- DE-A1-102008 036 934
- DE-A1-102010 007 559
- DE-A1-102010 012 162

## Beschreibung

Die Erfindung betrifft eine Vorrichtung zur Halterung einer bilderfassenden Einrichtung an einem Bioreaktor sowie einen Bioreaktor mit einer Vorrichtung zur Halterung einer bilderfassenden Einrichtung.

Verfahren und Vorrichtungen zur Herstellung biologischen Materials, wie beispielsweise biotechnologische Produktionsprozesse, welche die Kultivierung von Mikroorganismen, tierischen und pflanzlichen Zellen umfassen, sind von zunehmender Bedeutung. Biotechnologische Produktionsprozesse umfassen beispielsweise die Kultivierung von Mikroorganismen, tierischen und pflanzlichen Zellen. Das Monitoring beziehungsweise die Überwachung des Zellwachstums und der Vitalität von Zellen innerhalb des Bioreaktors ist dabei jedoch entscheidend für die Entwicklung und Steuerung von biotechnologischen Produktionsprozessen. Hierdurch wird die Produktausbeute und damit die Wirtschaftlichkeit des gesamten Prozesses beeinflusst.

In der Regel werden herkömmlich zur Bestimmung des Zellwachstums und der Vitalität der Zellen Proben dem Bioreaktor entnommen und diese dann außerhalb des Bioreaktors beziehungsweise off-line analysiert. Dadurch steht jedoch keine Prozesskontrolle in Echtzeit zur Verfügung. Standardverfahren umfassen hierbei Trübungsmessungen oder die Verwendung von Zählkammern. Die Trübungsmessung erfolgt regelmäßig anhand Messung der optischen Dichte. Hierbei wird eine Probe, gegebenenfalls verdünnt, in eine Küvette pipettiert und photometrisch bei, beispielsweise einer Wellenlänge von 880 nm, gemessen. Nachteile sind hierbei das erhöhte Kontaminationsrisiko durch den Probenzug und die unspezifische Messung aller bei der Messwellenlänge absorbierenden Stoffe. So suggeriert beispielsweise ein erhöhter Extinktionskoeffizient lysierter Zellen in fataler Weise ein Zellwachstum, obwohl sich die Zellen in Wirklichkeit in der Absterbe-Phase befinden. Ebenso werden bei derartigen Messungen Infektionen nicht erkannt. Die Folge kann hierbei sogar ein totaler Verlust der betroffenen Charge sein. Um die tatsächliche Vitalität der Zellen zu bestimmen, wird bisher eine sehr zeitaufwändige Untersuchung mit einem Mikroskop und einem Objektträger mit Zählkammer vorgenommen. Darüber hinaus werden häufig Anfärbungen mit beispielsweise Trypanblau vorgenommen. Dem hierbei entstehenden Zeitaufwand kann zwar mit kostenintensiver instrumenteller Analytik begegnet werden, beispielsweise einem Durchflusszytometer oder Coulter-Zähler, nachteilig bleibt jedoch das erhöhte Kontaminationsrisiko und die fehlende Bestimmung in Echtzeit.

Daten aus dem Inneren des Bioreaktors können zwar sterilisierbare Trübungssonden bereitstellen, aber auch diese weisen durch das Messverfahren bedingt die vorstehend beschriebenen Nachteile einer Trübungsmessung auf.

DE 10 2010 063 031 A1 zeigt einen potentiometrischen Sensor sowie ein Verfahren zur Inbetriebnahme eines potentiometrischen Sensors. Um den Einsatz eines als Einweg-Fermenter oder Einweg-Bioreaktor dienenden Behälters besonders einfach zu gestalten, kann die dort offenbarte potentiometrische Sonde bereits vor der Sterilisation, beispielsweise durch Bestrahlung mit Gammastrahlung, über einen Anschluss fest in eine Wand des Behälters eingebaut werden und für die Dauer der Lagerung und Verwendung darin verbleiben.

DE 10 2010 012162 A1 offenbart ein Verfahren zum Überwachen des Prozessablaufs in einem Bioreaktor und eine Überwachungsvorrichtung mit einer außerhalb des Bioreaktors angeordneten Kamera.

In DE 10 2008 036934 A1 wird ein Bioreaktor mit einem Fenster beschrieben, bei welchem die Standzeit von optischen Fenstern in Bioreaktoren verlängert werden soll, um möglichst ohne Unterbrechung des Betriebsvorganges für eine zeitraubende Reinigung der medienberührten Seite des Fensters auszukommen. Zu diesem Zweck soll eine Beschichtung des optischen Fensters verbessert werden.

WO 2018/014984 A1 zeigt eine Spektroskopiezelle in oder an einer Außenwandung eines Behälters sowie ein Spektroskopieverfahren, bei welchem die Spektroskopiezelle verwendet wird.

In dem Dokument DE 10 2006 022 307 A1 wird ein Einwegbioreaktor mit reversibel, äußerlich anbringbarer Sensoranordnung zur Messung einer physikalischen Größe eines enthaltenen Mediums beschrieben, wobei in wenigstens einer dem Zu- und/oder Abfluss von Medium dienenden Peripherieleitung des Bioreaktors ein Sensoradapter zur Aufnahme einer über eine innere Grenzfläche des Sensoradapters mit die Peripherieleitung durchströmendem Medium wechselwirkenden, elektronischen Sensoranordnung integriert ist. Da die Messung jeweils nur in der Peripherieleitung des Bioreaktors vorgenommen werden kann, ist eine Prozesskontrolle innerhalb des Bioreaktors mit dieser Anordnung nicht möglich.

DE 10 2010 037 923 A1 offenbart eine Bioreaktor-Anordnung für Zellen enthaltend einen abgeschlossenen Bioreaktor, einen Zellpelletträger zur Aufnahme eines Zellpellets und Mittel zum Zuführen von Nährlösung in das Zellpellet. Diese Anordnung erlaubt die kontaktlose Messung des Sauerstoffgehalts. Mit einem Laser werden hierbei Sauerstoffsonden zur Phosphoreszenz angeregt. Das emittierte Phosphoreszenzsignal wird mit dem Detektor aufgenommen und an eine Auswerteelektronik geleitet. Hierzu weist der Bioreaktor lichtdurchlässige Fenster auf und der Laser und der Detektor sind außerhalb des Bioreaktors angeordnet. Ein Austausch von Sensoren oder Messeinrichtungen wird in diesem Dokument nicht beschrieben.

DE 10 2011 101 108 A1 beschreibt eine Transflexionssonde zur Durchführung einer Transflexionsmessung an einem in einem starren Behälter befindlichen Fluid mit einen mit einer Lichtleitstrecke in seinem Inneren ausgestatteten Sondenschaft, an dessen vorderer Stirnseite eine offene Durchflusskammer mit einer der vorderen Stirnseite des Sondenschaftes gegenüberliegenden Reflexionsscheibe angeordnet ist. Der Sondenschaft ist als ein an seiner vorderen Stirnseite von einem transparenten Fenster abgeschlossener, starrer Hohlkörper ausgebildet, der an seinem rückwärtigen Ende eine erste Koppeleinrichtung zur starren Ankopplung eines Sensormoduls an den Sondenschaft aufweist. Diese Koppeleinrichtung ist jedoch mit der offenen Durchflusskammer und insbesondere mit der der vorderen Stirnseite des Sondenschaftes gegenüberliegenden Reflexionsscheibe fest verbunden, sodass der Austausch von Sensormodulen an einer Sonde beziehungsweise die wechselnde Ankopplung eines Sensormoduls an unterschiedliche Sonden desselben oder verschiedenen Behälter ermöglicht wird. Ein Austausch von Sensoren zur Messung physikalischer, chemischer oder biologischer Messgrößen wird nicht offenbart.

DE 10 2011 117 228 A1 betrifft ein Mikroskopiesystem zur Bestimmung des Zustands von biologischen Zellen, das eine optische Bilderfassungseinheit mit einem optischen Sensor und eine optionale Beleuchtungsvorheit aufweist. Die optische Bilderfassungseinheit ist von einer wasser- und feuchtedichten Umhüllung umschlossen, die über einer Sensorfläche des optischen Sensors oder zumindest einem Bereich dieser Sensorfläche durch eine optisch transparente Schicht gebildet ist. Durch die Übertragung der aufgenommenen Bilddaten von Zellen, die an der optisch transparenten Schicht anhaften, an eine externe Empfangseinheit lässt das Mikroskopiesystem zur Bestimmung des Zustands von Zellen direkt in einen Bioreaktor einsetzen. Das Mikroskopiesystem besteht aus einem extrem stark miniaturisierten Mikroskop, einer drahtlosen Datenübertragungseinheit, einer Energieversorgungseinheit, einem geeigneten wasser- und feuchtedichten und sterilisierbaren Gehäuse und einer außerhalb des Bioreaktors befindlichen Empfangseinheit. Nachteilig ist an diesem Mikroskopiesystem die Anordnung des Mikroskops innerhalb des zu untersuchenden Mediums, welches wegen der notwendigen Energieversorgung die Betriebsdauer begrenzt, da zur Bilderfassung ein CCD-Chip verwendet wird, der als miniaturisierte bilderfassende und bildgebende Einheit eingesetzt wird. Darüber hinaus ist während beispielsweise eines Produktionsprozesses ein Eingriff an diesem Mikroskop und ist auch dessen Austausch nicht möglich. Durch die Verwendung eines CCD-Chips ist die optische Auflösung dieses Mikroskops durch die Pixelgröße der Photosensoren des Chips beschränkt.

Der Erfindung liegt die Aufgabe zugrunde, bilderfassende und bevorzugt bildgebende Systeme derart auszugestalten, dass diese austauschbar an einem Bioreaktor anbringbar sind und dabei Bilddaten eines innerhalb des Bioreaktors befindlichen Volumens zumindest erfassen können, vorzugsweise jedoch auch bildgebend verarbeiten oder weitergeben können.

Die Erfassung von Bildinformationen, insbesondere bei bilderfassenden Einrichtungen oder Systemen bezeichnet im Rahmen dieser Offenbarung die Aufnahme von elektromagnetischen Wellenfeldern, insbesondere von Wellenfeldern innerhalb des Behälters eines Bioreaktors in einem Spektralbereich mit Wellenlängen von 250 bis 2000 nm in einer optischen Einrichtung, insbesondere in einer mikroskopischen Einrichtung wie beispielsweise einem Mikroskop, oder auch die Aufnahme von elektromagnetischen Wellenfeldern, insbesondere in einem Spektralbereich mit Wellenlängen von 250 bis 2000 nm in elektrooptischen Einrichtungen, wie beispielsweise in einzelnen Photosensoren oder auch in einem Array angeordneten Photosensoren, wie in CCD- oder CMOS-Sensoren die entweder eine zeilenweise oder eine flächige Anordnung von Photosensoren aufweisen, welche dann auch im Rahmen dieser Offenbarung als Pixel bezeichnet werden, umfassen. Diese elektrooptischen Einrichtungen können Teil eines optischen Systems sein oder auch direkt das jeweilige elektromagnetische Wellenfeld aufnehmen. Das jeweilige Wellenfeld kann zeitlich kontinuierlich oder auch in Form von Bursts, somit zeitlich begrenzten Pulsen oder Lichtpulsen vorliegen, welche dann auch als stroboskopisch emittierte Wellenfelder bezeichnet werden und in zeitlich definierter Folge vorliegen und erfasst werden können.

Als bildgebende Verarbeitung wird hierbei sowohl die Beeinflussung optischer Strahlengänge, wie dies beispielsweise in mikroskopischen Einrichtungen der Fall ist, bezeichnet, als auch die Wandlung elektrischer Signale, insbesondere optoelektrischer Signale in ein Datenformat, bezeichnet, welches Bilddaten, insbesondere auch digitale Bilddaten enthält.

Diese Aufgabe wird mit dem Gegenstand der unabhängigen Ansprüche gelöst. Bevorzugte Weiterbildungen sind den abhängigen Ansprüchen sowie der Beschreibung zu entnehmen.

Vorzugsweise soll dabei der Abstand zu dem im Bioreaktor befindlichen fluiden Medium zur bilderfassenden Einheit, insbesondere zu einer mikroskopischen Einrichtung, die optische Erfassung nur wenig beeinträchtigen oder kann die erfindungsgemäße Vorrichtung bei bevorzugten Ausführungsformen auch ein strahlformender Teil einer bilderfassenden Einrichtung sein.

Gegenüber dem Stand der Technik wird mit der Erfindung eine effektive in-situ Prozesskontrolle bewirkt. Für die Herstellung von Biopharmazeutika wird beispielsweise ein geringeres Produktionsrisiko und eine höhere Ausbeute dauerbetriebsfest ermöglicht, ohne hierbei einen kostenintensiven Produktionsprozess aufgrund einer mangelnden Energieversorgung unterbrechen zu müssen.

Gegenüber dem Stand der Technik haben die bevorzugten Ausführungsforen den weiteren Vorteil, dass der Abstand zwischen einer bilderfassenden Einheit und fluiden Medium, insbesondere auch zu dem darin enthaltenen biologischen Material variabel und flexibel, selbst während der Kultivierung, verändert werden kann. Die komplette Optikeinheit und auch die Bilderfassung samt Anzahl und Abstand sämtlicher optischer Linsen sowie Kontrastsysteme sind zu jedem Zeitpunkt flexibel ohne ein zusätzliches Kontaminationsrisiko austauschbar.

Durch die mechanische Trennung der zu sterilisierenden Baugruppe von der mikroskopischen Einrichtung, können Anforderungen an die Sterilität auf der einen und an die Materialwahl der mikroskopischen Einrichtung auf der anderen Seite unabhängig voneinander effizienter erfüllt werden. Durch die Erfindung entfallen somit beispielsweise auch für herkömmliche Mikroskopsonden die Materialanforderungen wegen Sterilisierbarkeit und eventuelle Zulassungsbeschränkungen, wodurch sich nicht nur konstruktive, sondern auch Kostenvorteile ergeben.

Der Sterilbereich innerhalb des Behälters zur Aufnahme fluider Medien, die biologisches Material enthalten, des jeweiligen Bioreaktors wird vom Bereich außerhalb des Bioreaktors abgetrennt und mikroskopische Einrichtungen, wie beispielsweise Mikroskopsonden, können mechanisch stabil gehalten werden. Die Erfindung ist sowohl bei Einwegbioreaktoren, welche auch als Single-Use-Reaktoren bezeichnet werden, als auch bei für die Mehrfachnutzung vorgesehenen Bioreaktoren, die fachüblich auch Multi-Use-Reaktoren genannt werden, anwendbar.

Ein erfindungsgemäßer Bioreaktor umfasst einen Behälter zur Aufnahme von fluiden Medien, die biologisches Material enthalten, eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten, eine Vorrichtung zur Halterung einer bilderfassenden Einrichtung, insbesondere einer mikroskopischen Einrichtung, bei welcher eine Halterung für die bilderfassende Einrichtung ein Fenster mit einem transparentem Element, das für elektromagnetische Strahlung transparent ist, aufweist, wobei die Vorrichtung zur Halterung einer bilderfassenden Einrichtung zumindest teilweise in der Durchgangsöffnung der Durchführung gehalten ist, wobei das Fenster die Durchgangsöffnung verschließt. Hierbei soll der Ausdruck, dass das Fenster die Durchgangsöffnung verschließt auch umfassen, dass die Vorrichtung zur Halterung einer bilderfassenden Einrichtung zusammen mit deren Fenster die Durchgangsöffnung verschließt.

Bevorzugt umfasst die Durchführung einen Standardport, wie beispielsweise einen Ingold-Port, einen Broadly-James-Port, einen B.-Braun-Sicherheitsport oder einen einem anderen Standard entsprechenden Port. Bei Einwegreaktoren oder Single-Use-Reaktoren kann die Durchführung auch einen Tri-Clamp-, Sanitary-Clamp- oder einen herstellerspezifischen, insbesondere herstellerspezifisch angepassten Port umfassen.

Zusätzlich kann insbesondere bei einem Single-Use-Reaktor eine Befestigung mit externem Haltemitteln, wie beispielsweise einem Schnappverschluss vorgesehen sein.

Diese weisen jeweils eine Durchgangsöffnung definierten Durchmessers auf, welche in typischer Weise das Innere eines Bioreaktors mit dessen Äußerem verbindet oder eröffnet.

Generell kann der Behälter zur Aufnahme von fluiden Medien, die biologisches Material enthalten, der Behälter eines Multi-Use-Bioreaktors zur Mehrfachanwendung sein.

Vorteilhaft umfasst der Behälter zur Aufnahme von fluiden Medien, die biologisches Material enthalten, dann Edelstahl oder besteht dieser aus Edelstahl. Auch die Vorrichtung zur Halterung einer bilderfassenden Einrichtung an einem Bioreaktor kann Edelstahl umfassen oder es können zumindest eines oder mehrere von deren Bestandteilen sowie der Grundkörper der hier offenbarten Fenster aus Edelstahl bestehen.

Verwendbar sind hierbei alle Edelstähle, insbesondere auch austenitische und ferritische Edelstähle, bevorzugt jedoch nur soweit diese bei der Durchführung der Erfindung rostfrei bleiben.

Der Edelstahl kann bevorzugt auch 316L Pharmastahl umfassen, oder vollständig aus diesem bestehen.

Ferner sind prinzipiell auch Titan, Tantal, Niob, deren Legierungen, sowie Monell-Legierung mit hohem Kupferanteil verwendbar, wobei das Material bei Verwendung für die Beleuchtungsvorrichtung, welche eine Vorrichtung zur Abgabe elektromagnetischer Strahlung darstellt, insbesondere für deren Gehäusekörper auch emailliert sein kann.

Alternativ kann der Behälter zur Aufnahme von fluiden Medien, die biologisches Material enthalten, auch der Behälter eines Single-Use-Bioreaktors zur Einweganwendung sein.

Vorteilhaft ist es in diesem Fall, wenn der Behälter zur Aufnahme von fluiden Medien, die biologisches Material enthalten, einen Kunststoff, insbesondere einen sterilisierbaren Kunststoff umfasst oder aus einem Kunststoff, insbesondere einem sterilisierbaren Kunststoff besteht. Dieser Kunststoff kann ein polymeres Material umfassen und kann insbesondere aus einem geeigneten Material bestehen, welches eine Gamma- bzw. eine chemische Sterilisierung mit ETO übersteht.

Um den strengen Anforderungen für die Herstellung von Biopharmazeutika zu genügen, kann das Material, welches der Bioreaktor und welches die Sensoraufnahme jeweils umfassen so ausgewählt sein, dass dieses jeweils den nachfolgenden Standards entspricht:
i) FDA approved materials (ICH Q7A, CFR 211.65(a) - Code of Federal Regulations, USP Class, animal derivative free, bisphenol A free)
ii) EMA (European Medicines Agency) EU GMP Guide Part II approved materials
iii) Sectoral chemical resistance - ASTM D 543-06 Biocompatibility e.g. referred to US Pharmacopeia or tests referred to ISO 10993.

Besonders vorteilhaft ist es, wenn der Bioreaktor zusammen mit der Vorrichtung zur Halterung einer bilderfassenden Einrichtung autoklavierbar ist, insbesondere während diese zumindest teilweise in der Durchgangsöffnung der Durchführung gehalten ist, autoklavierbar ist, denn dann kann hierdurch eine Kontamination mit biologisch aktivem oder wechselwirkendem Material mit sehr hoher Sicherheit ausgeschlossen werden.

Hierzu ist insbesondere auch die Vorrichtung zur Halterung einer bilderfassenden Einrichtung autoklavierbar ausgebildet.

Überraschend hat es sich gezeigt, dass bei den hier offenbarten Vorrichtungen zur Halterung einer bilderfassenden Einrichtung 3500 Auktoklavierungs-Zyklen bei 2 bar und 134°C möglich waren.

Autoklavierbar wird im Rahmen dieser Offenbarung auch als autoklavierbar im Sinne der DIN EN ISO 14937; EN ISO 17665, welche für Medizinprodukte gültig ist, verstanden.

Mit einem Fenster mit einem transparenten Element, das für elektromagnetische Strahlung transparent ist sowie einer Halterung für eine bilderfassende Einrichtung, kann der Sterilbereich zuverlässig geschützt werden, auch wenn während eines Produktionsprozesses bilderfassende oder bildgebende Einrichtungen ausgetauscht werden.

Eine mechanisch stabile Anordnung ergibt sich, durch einen Grundköper, an welchem das transparente Element des Fensters hermetisch dicht, vorzugsweise mittels einer Druckverglasung, insbesondere einer GTMS-Druckverglasung angeordnet ist.

Die mechanische Stabilität wird weiter unterstützt, wenn ein Halterungskörper der Vorrichtung zur Halterung einer bilderfassenden Einrichtung zylindersymmetrisch, insbesondere säulenförmig ausgebildet ist und eine Durchgangsöffnung aufweist, welche auf der dem Inneren des Bioreaktors zugeordneten Seite mittels des transparenten Elements des Fensters fluiddicht, insbesondere hermetisch dicht abgeschlossen ist. Da eine mikroskopische Einrichtung in diesem Fall sogar in direkten Kontakt mit dem transparenten Element des Fensters treten kann, werden hohe numerische Aperturen für bildgebende Systeme ermöglicht, welche eine weitaus bessere optische Abbildungsleistung bereitstellen können, als dies bisher möglich war.

Vorteilhaft kann das transparente Element des Fensters in einem Spektralbereich mit einer Wellenlänge von 250 bis 2000 nm eine Transmission aufweist, die höher ist als 80%, besonders bevorzugt höher als 90 %.

Bei einer besonders bevorzugten Ausführungsform umfasst das transparente Element des Fenster Glas oder besteht aus Glas.

Bevorzug kann das Glas des transparenten Elements des Fensters Quarzglas oder Borosilikatglas umfassen.

Optische Vorteile können sich für bilderfassende und bildgebende Systeme ergeben, wenn das transparente Element des Fensters scheibenförmig ausgebildet ist und insbesondere planparallele Hauptoberflächen aufweist. Insbesondere ist es bei Mikroskopoptiken bekannt, beispielsweise für die Verwendung von planparallelen Abdeckplatten planparallele optische Strahlbeeinflussungen zu korrigieren und dadurch dennoch zu optischen Höchstleistungen zu gelangen. Derartige optische Korrekturen können auch bei dieser bevorzugten Ausführungsform insbesondere mit einer vorteilhaft verbesserten Distorsionsfreiheit Verwendung finden.

Wenn jedoch das transparente Element des Fensters plankonvex, plankonkav, bikonvex, bikonkav, konvexkonkav oder konkavkonvex geformt ist, kann dieses sogar selbst Teil eines abbildenden Systems sein.

Bevorzugt kann dabei das transparente Element des Fensters Teil einer diesem zugeordneten mikroskopischen Einrichtung sein.

Der Halterungskörper kann einen seitlichen, sich in radialer Richtung erstreckenden Absatz aufweisen, um insbesondere damit einen Anschlag in axialer Richtung an einem am Bioreaktor angeordneten Standardport auszubilden.

Dann kann bei in die Durchgangsöffnung eingesetzter Vorrichtung zur Halterung einer bilderfassenden Einrichtung der axiale Abstand des transparenten Elements des Fensters zur Innenwand des Bioreaktors durch den axialen Abstand des transparenten Elements des Fensters zum seitlichen Absatz definiert werden. Dann ist das transparente Element des Fensters bei in die Durchgangsöffnung eingesetztem Halterungskörper, insbesondere bei Anlage des seitlichen Absatzes an einem Standardport vorteilhaft innerhalb des Bioreaktors angeordnet. Ein Satz von Halterungskörpern mit verschiedenem axialem Abstand des transparenten Elements des Fensters zum seitlichen Absatz kann hierbei eine wählbare axiale Positionierung des transparenten Elements des Fensters innerhalb des Bioreaktors ermöglichen. Als axialer Abstand wird im Rahmen dieser Offenbarung ein Abstand verstanden, der in Richtung der Symmetrieachse des zylindersymmetrischen Halterungskörpers der Sensoraufnahme jeweils gemessen oder angegeben ist.

Sehr vorteilhaft ist eine Beleuchtungsvorrichtung, welche an der dem Inneren des Behälters zur Aufnahme fluider Medien zugeordneten Seite am Halterungskörper angeordnet, vorzugsweise lösbar befestigbar angeordnet ist, denn mit dieser können definierte Beleuchtungsverhältnisse für das fluide Medium oder die fluiden Medien bereitgestellt und nachfolgend in der bilderfassenden Einrichtung, insbesondere mikroskopischen Einrichtung erfasst werden.

Bevorzugt umfasst die Beleuchtungsvorrichtung ein Gehäuse und ein weiteres Fenster, vorzugsweise mit einem weiteren Grundkörper und einem weiteren transparenten Element, welches dem Fenster, insbesondere dem transparenten Element des Fensters der Halterung der Vorrichtung zur Halterung einer bilderfassenden Einrichtung im montierten Zustand gegenüberliegt. Hierdurch wird ein definiertes Beleuchtungsfeld bereitgestellt, welches eine definierte Bilderfassung ermöglicht.

Als montierter Zustand wird im Sinne dieser Offenbarung derjenige Zustand bezeichnet, bei welchem die Beleuchtungsvorrichtung am Halterungskörper angebracht, vorzugsweises lösbar angebracht ist, und deren Betriebsstellung einnimmt, wie dies nachfolgend noch detaillierter beschrieben werden wird.

Weitere Vorteile ergeben sich auch, wenn das transparente Element des Fensters, welches am Halterungskörper der Vorrichtung zur Halterung einer bilderfassenden Einrichtung angeordnet ist, mittels einer Druckverglasung, insbesondere GTMS-Druckverglasung am Grundkörper und dieser vorzugsweise durch eine Schweißverbindung am Halterungskörper gehalten ist und/oder das transparente Element des Fenster der Beleuchtungsvorrichtung mittels einer Druckverglasung, insbesondere GTMS-Druckverglasung am Grundkörper und dieser durch eine Schweißverbindung am Gehäuse der Beleuchtungsvorrichtung gehalten ist, denn dann, wird jeweils eine mechanisch sowie thermisch stabile Verbindung bereitgestellt, welche auch gegenüber Autoklavierung sowie einer Reinigung und Sterilisierung mittels der als CIP (Clean-In-Place) und als SIP (Sterilization-In-Place) bezeichneten Verfahren dauerhaft und betriebssicher beständig ist.

Das mikroskopseitige transparente Element des Fensters am Grundkörper, insbesondere GTMS-Fenster (Glass-to-Metal-Seal-Fenster) wird vorzugsweise nach der Druckeinglasung geschliffen und poliert. Dies ermöglicht eine besonders verzerrungsfreie Bilderfassung und -aufnahme.

Das Beleuchtungsvorrichtungs-seitige transparente Element des Fensters, insbesondere das GTMS-Fenster ermöglicht eine optimierte Ausleuchtung für die jeweilige Bilderfassung.

Besonders bevorzugt ist dabei der Grundkörper des Fensters der Vorrichtung zur Halterung einer bilderfassenden Einrichtung durch eine Schweißverbindung, insbesondere mittels Schweißen, insbesondere Laser-Schweißen hermetisch dicht mit dem Halterungskörper verbunden und ist optional auch der Grundkörper des Fensters der Beleuchtungsvorrichtung durch eine Schweißverbindung, insbesondere mittels Schweißen, insbesondere Laser-Schweißen hermetisch dicht mit dem Gehäuse der Beleuchtungsvorrichtung verbunden.

Als GTMS-Druckverglasung wird im Rahmen der vorliegenden Offenbarung eine dem Fachmann an sich bekannte Druckverglasung einer Glas umfassenden Scheibe, insbesondere einem entsprechenden transparenten Element mit einem diese oder dieses umgebenden Metall verstanden. Hierbei kann eine kreisförmige Scheibe innerhalb eines Metallrings angeordnet sein, an welchen diese bei erhöhter Temperatur angeschmolzen und durch nachfolgende Temperaturabsenkung unter Druck fluiddicht und insbesondere hermetisch dicht gehalten ist.

Generell kann durch Schweißen, insbesondere Laserschweißen, insbesondere nach der Druckeinglasung vorteilhaft eine weitere fluid- und hermetisch dichte Verbindung beispielsweise von dem Grundkörper der Druckeinglasung zum Halterungskörperköper oder von dem Grundkörper der Druckeinglasung des weiteren Fensters zum Gehäuse einer Beleuchtungsvorrichtung hergestellt werden.

Vorteilhaft kann die Vorrichtung zur Halterung einer bilderfassenden Einrichtung und deren Beleuchtungsvorrichtung im montierten Zustand innerhalb des Behälters zur Aufnahme von fluiden Medien eine Kammer mit einem definierten, insbesondere zu zumindest zwei Seiten senkrecht zur Symmetrieachse des Halterungsköpers hin offenem Volumen ausbilden. Hierdurch kann eine definierte Messkammer innerhalb des Behälters zur Aufnahme fluider Medien bereitgestellt werden, mit welcher auch Trübungsmessungen unter definierten Randbedingungen und in Echtzeit ermöglicht werden. Insbesondere können diese Messungen alternativ oder parallel zur jeweiligen Bilderfassung durchgeführt werden. Hierzu können darüber hinaus auch Bilddaten, welche von der bilderfassenden Einrichtung an eine bildgebende Einrichtung weitergeleitet wurden entsprechend ausgewertet werden.

Vorteilhaft können dabei oder generell bei sämtlichen Ausführungsformen das transparente Element des Fensters der Beleuchtungsvorrichtung und das transparente Element des Fensters, welches am Halterungskörper der Vorrichtung zur Halterung einer bilderfassenden Einrichtung angeordnet ist, einen definierten Abstand zueinander aufweisen.

Bei einer besonders bevorzugten Ausführungsform kann die Vorrichtung zur Halterung einer bilderfassenden Einrichtung an deren Halterungskörper zumindest einen elektrischen Verbinder und auch die Beleuchtungsvorrichtung an deren Gehäuse zumindest einen elektrischen Verbinder aufweisen, welche miteinander fügekompatibel und jeweils hermetisch abgedichtet sind, wodurch vorzugsweise eine zum Bioreaktor externe Ansteuerung der Beleuchtungsvorrichtung ermöglicht wird. Bei einer bevorzugten Ausführungsform weist diese hermetische Abdichtung, insbesondere bei steckbarer Ausführung ein Gehäuse eines jeweiligen Steckers auf, welches mit dem Halterungskörper oder mit einem Sockelteil jeweils über eine Elastomer Komponente gedichtet ist. Zugelassen sind hierfür beispielsweise Werkstoffe wie EPDM, NBR oder FKM.

Alternativ oder zusätzlich kann Vorrichtung zur Halterung einer bilderfassenden Einrichtung an deren Halterungskörper zumindest einen induktiven Koppler und die Beleuchtungsvorrichtung an deren Gehäuse zumindest einen induktiven Koppler aufweisen, welche miteinander im montierten Zustand induktiv koppelbar und jeweils hermetisch abgedichtet sind.

Als hermetisch dicht oder auch fluiddicht wird im Sinne der vorliegenden Offenbarung wird ein Gegenstand oder auch eine Verbindung zwischen zwei Gegenständen, beispielsweise zwischen dem transparentem Element 12 und Grundkörper 10 des Fensters 11, dann angesehen werden, wenn diese bei Raumtemperatur eine Leckrate von weniger als 1x10-3 mbar * l/sec aufweisen, wenn diese einseitig mit He und 1 bar Druckdifferenz beaufschlagt werden.

Bevorzugt umfasst die Beleuchtungsvorrichtung eine LED oder einen Array von LEDs, die jeweils in dem Gehäuse der Beleuchtungsvorrichtung angeordnet sind.

Die LEDs, welche auch OLEDs sein können, sind bei einer besonders bevorzugten Ausführungsform mittels einer zugeordneten elektronischen Einrichtung, insbesondere einer Prozesssteuerungseinrichtung (PST), stroboskopisch ansteuerbar, insbesondere auch, um Unschärfe durch Bewegungen des biologischen Materials innerhalb des fluiden Mediums oder der fluiden Medien zu mildern oder bei entsprechend kurzen, Licht-abgebenden Pulsen oder Bursts sogar zu vermeiden.

Auf diese Weise können auch scharfe, automatisiert auswertbare Bilder des durch das Rührorgan in Bewegung befindlichen fluiden Mediums, insbesondere einer Zellsuspension aufgenommen werden.

Vorteilhaft ist dabei eine getaktete und mit einer jeweiligen Aufnahme synchronisierte Auslösung eines LED-Blitzes oder Burst.

Im Rahmen der vorliegenden Offenbarung werden die Begriffe Licht und elektromagnetische Strahlung synonym gebraucht. Es soll jedoch der Begriff der elektromagnetischen Strahlung entsprechend offenbaren, dass die vorliegende Erfindung nicht auf den Spektralbereich des sichtbaren Lichts beschränkt ist, wie dieses beispielsweise auch an den Transmissionseigenschaften der hier offenbarten Gläser zu erkennen ist.

Ferner können bei einer stroboskopischen Beleuchtung zeitsynchrone oder auch zeitlich asynchrone Abgaben von Licht vorliegen wobei sich der Begriff der synchronen Abgabe von Licht auf eine synchrone Bilderfassung mittels der mikroskopischen Einrichtung bezieht. Hierbei können synchrone Aufzeichnungen zur Verminderung von Unschärfen verwendet werden und beispielsweise asynchrone Abgaben von elektromagnetischer Strahlung, mit einer beispielsweise zeitlich versetzten, nachfolgenden Bildaufzeichnung ein "Nachleuchten" des biologischen Materials oder des fluiden Mediums erfassen, welches zur Überwachung von Stoffwechselvorgängen des biologischen Materials verwendbar ist.

Bevorzugt umfasst die Erfindung auch ein Verfahren zur Vermehrung oder Kultivierung biologischen Materials umfassend das Einbringen von fluiden Medien, die biologisches Material oder eine Vorstufe biologischen Materials enthalten in einen Bioreaktor, insbesondere wie dieser vorliegend offenbart wird, insbesondere in einen Behälter des Bioreaktors zur Aufnahme von fluiden Medien, die biologisches Material enthalten, wobei der Behälter zur Aufnahme von fluiden Medien, die biologisches Material enthalten, eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten aufweist, und eine Vorrichtung zur Halterung einer bilderfassenden Einrichtung, insbesondere einer mikroskopischen Einrichtung mit einer Halterung für die bilderfassende Einrichtung, welche ein Fenster mit einem transparenten Element, das für elektromagnetische Strahlung transparent ist, umfasst, an oder in der Durchführung des Behälters zur Aufnahme von fluiden Medien, die biologisches Material enthalten, vor dem Einbringen der fluiden Medien, die biologisches Material oder eine Vorstufe biologischen Materials enthalten angebracht wird.

Besonders bevorzugt kann das Verfahren zur Vermehrung oder Kultivierung die Herstellung von Pharmazeutika, insbesondere von Biopharmazeutika umfassen. Hierbei umfasst der Begriff der Kultivierung auch die Präparation des biologischen Materials, dies bedeutet auch dessen Veränderung, sogar Schädigung, denn es können beispielsweise über zelluläre Reparaturmechanismen erhöhte Erträge bei der Vermehrung biologischen Materials erbracht werden.

Ein besonderer Vorteil ergibt sich, wenn der Bioreaktor nach dem Anbringen der Vorrichtung zur Halterung einer bilderfassenden Einrichtung, insbesondere einer mikroskopischen Einrichtung, sterilisiert wird, denn dann kann unabhängig von einer in der Vorrichtung zur Halterung einer bilderfassenden Einrichtung angeordneten bilderfassenden Einrichtung oder auch unabhängig von einem Austausch einer in der Vorrichtung zur Halterung einer bilderfassenden Einrichtung angeordneten Vorrichtung stets ein steriler Bioreaktor bereitgestellt und das Risiko von Kontaminationen vermindert werden.

Vorteilhaft kann nach Anbringen der Vorrichtung zur Halterung einer bilderfassenden Einrichtung diese mit einer bilderfassenden Einrichtung bestückt werden und kann das Erfassen von Bildinformationen mit der bilderfassenden Einrichtung vorgenommen werden.

Besonders vorteilhaft kann von einer Beleuchtungsvorrichtung, welche vorzugsweise an der dem Inneren des Behälters zur Aufnahme fluider Medien zugeordneten Seite am Halterungskörper angeordnet, vorzugsweise lösbar befestigbar angeordnet ist, elektromagnetische Strahlung abgegeben, insbesondere in den Behälter zur Aufnahme fluider Medien eingestrahlt werden.

Dabei kann bei einer bevorzugten Ausführungsform von der Beleuchtungsvorrichtung elektromagnetische Strahlung in Form von zeitlich beschränkten Pulsen abgegeben werden.

Die zeitlich beschränkten Pulse können beispielsweise jeweils pro Puls eine Zeitdauer von 1 ms bis weniger als ein Tausendstel einer µs aufweisen.

Die zeitlich beschränkten Pulse der elektromagnetischen Strahlung können in einer zeitsynchronen Weise und somit in festen Zeitintervallen oder auch in zeitlich asynchrone Weise und somit in variierenden Zeitintervallen abgegeben werden.

Die zeitlich beschränkten Pulse der elektromagnetischen Strahlung in einer zeitsynchronen Weise abgegeben werden, bedeutet dies im Sinne der vorliegenden Offenbarung, dass diese Pulse bevorzugt mit einer festen Frequenz abgegeben werden.

Diese festen Frequenzen können bei einem Hz bis zu mehreren MHz liegen, insbesondere, wenn zur Abgabe der zeitlich beschränkten Pulse Laser-Leds verwendet werden.

Besonders voreilhaft kann das Erfassen von Bildinformationen mit der bilderfassenden Einrichtung während eines zeitlich beschränkten Intervalls synchron zur Abgabe der elektromagnetische Strahlung in Form von zeitlich beschränkten Pulsen erfolgen.

Bei einer weiteren besonders bevorzugten Ausführungsform umfasst die Vorrichtung zur Halterung einer bilderfassenden Einrichtung zumindest einen Sensor und kann im Betriebszustand die Strahlungsintensität und/oder Wellenlänge der elektromagnetischen Strahlung im Innern des Bioreaktors gemessen werden.

Hierbei kann besonders bevorzugt im Betriebszustand die Strahlungsintensität und/oder Wellenlänge der elektromagnetischen Strahlung im Innern des Bioreaktors ortsaufgelöst gemessen werden, beispielsweise mittels einer bilderfassenden Einrichtung wie etwa einer Mikroskopsonde.

Wenn für einen definierten Zeitraum elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird, können hierdurch fluoreszent emittierte Anteile des Lichts detektiert und bezüglich definierter Stoffwechselvorgänge innerhalb des Bioreaktors ausgewertet werden.

Besonders bevorzugt werden bei den hier beschriebenen Verfahren auch durch Mutagenese veränderte photo- oder mixotrophen Mikroorganismen, insbesondere auch Mikroalgen, Hefen und Bakterien, verwendet.

Mit der Erfindung sowie deren bevorzugten Ausführungsformen wird eine Minderung des Kontaminationsrisikos erreicht, da insbesondere kein Probenzug mehr erfolgen muss, und eine Produktionskontrolle in Echtzeit erfolgen kann. Der Anwender kann während einer Kultivierung gefahrlos die bilderfassende Einrichtung, beispielsweise eine Mikroskopsonde wechseln und es entsteht kein Kontrollverlust mehr bei deren Defekt. Eine Überwachung beziehungsweise ein Monitoring des Zellwachstums inklusive der Erfassung der Morphologie, der Erfassung von vitalen und proliferierenden Zellen, der Erfassung von Fragmenten lysierter Zellen sowie von Infektionen und Kontaminationen wird dauerhaft ermöglicht und somit Ausbeutesteigerungen gefördert.

Sehr vorteilhaft ist ein Verfahren bei welchem der Bioreaktor mit dessen Behälter zur Aufnahme von fluiden Medien, die biologisches Material enthalten, und der Vorrichtung zur Halterung einer bilderfassenden Einrichtung autoklaviert wird während die Vorrichtung zur Halterung einer bilderfassenden Einrichtung zumindest teilweise in der Durchgangsöffnung der Durchführung gehalten ist, wobei vorzugsweise das Fenster die Durchgangsöffnung verschließt, denn dann können Kontaminationen des Photobioreaktors mit sehr viel höherer Wahrscheinlichkeit als bisher vermieden werden und kann die Autoklavierung mit einem einzigen Vorgang durchgeführt werden. Wird folglich die Autoklavierung zeitnahe vor der Befüllung des Photobioreaktors mit dem biologischen Material vorgenommen, reduziert sich auch das Risiko des zwischenzeitlichen Eintrags von Verschmutzungen.

Bei den hier offenbarten Verfahren können insbesondere auch durch Mutagenese veränderte photo- oder mixotrophen Mikroorganismen insbesondere auch Mikroalgen, Hefen und Bakterien, verwendet werden.
Durch die hier beschriebenen Ausführungsformen, insbesondere auch des Verfahrens, lassen sich Vorgänge innerhalb eines Bioreaktors sehr flexibel und mit hoher Präzision erfassen. Insbesondere sind auch durch die mikroskopische Erfassung sowie die

Der Artikel "Adhesion of Chlamydomonas microalgae to surfaces is switchable by light", Christian Titus Kreis, Marine Le Blay, Christine Linne, Marcin Michal Makowski and Oliver Bäumchen, NATURE PHYSICS DOI: 10.1038/NPHYS4258 beschreibt das Verhalten von Mikroalgen, welche mittels der Einstrahlung von Licht dazu gebracht werden konnten, sich an Oberflächen anzuhaften oder von Oberflächen zu lösen. Durch gezielte Einstrahlung elektromagnetischer Strahlung entsprechender Wellenlänge kann nun beispielsweise für Photobioreaktoren, welche im repeated Batch-Betrieb eingesetzt werden, erreicht werden, dass sich während des Wechsels eines Mediums, beispielsweise von Nährlösung, diese Mikroalgen an Oberflächen anhaften und nach Zufuhr des Mediums sich wieder von der Oberfläche lösen. Auch hierdurch kann die Effektivität des Betriebs von Photobioreaktoren weiter gesteigert werden. Ein derartiges Verhalten von Mikroalgen lässt sich beispielsweise mit den hier beschriebenen bilderfassenden Vorrichtungen überwachen.

Die Autoren Ni, M., Tepperman, J.M. and Quail, P.H. (1999) beschreiben in der Veröffentlichung (Phytochromes and gene expression), "Binding of phytochrome B to its nuclear signalling partner PIF3 is reversibly induced by light, Nature 400, 781-784, dass sich die Genexpression über die entsprechenden Zellsignalwege gezielt lichtabhängig induzieren lässt.

Die Veröffentlichung "A red/far-red light-responsive bistable toggle switch to control gene expression in mammalian cells", Konrad Müller, Raphael Engesser, Stephanie Metzger, Simon Schulz, Michael M. Kämpf, Moritz Busacker, Thorsten Steinberg, Pascal Tomakidi, Martin Ehrbar, Ferenc Nagy, Jens Timmer, Matias D. Zubriggen und Wilfried Weber, Nucleic Acids Research, 2013, Vol. 41, No. 7 e77, beschreibt diesen Mechanismus für Mammalia-Zellen, um transgene Aktivität zu kontrollieren. Optogenetische Kontrolle ist auch in beschrieben in "A Phytochrome-Derived Photoswitch for Intracellular Transport", Max Adrian, Wilco Nijenhuis, Rein I. Hoogstraaten, Jelmer Willems, and Lukas C. Kapitein, DOI: 10.1021/acssynbio.6b00333 ACS Synth. Biol. 2017, 6, 1248-1256. Wellenlängenspezifisch lassen sich gemäß diesem Dokument intrazelluläre bidirektionale Transportmechanismen steuern. Hierbei ist eine Prozesskontrolle mit bildgebenden Einrichtungen, auch als Signal für eine Rückkopplung in die Prozessteuerung äußerst hilfreich.

Die Erfindung wird nachfolgend anhand bevorzugter Ausführungsformen und unter Bezugnahme auf die nachfolgenden Zeichnungen detaillierter beschrieben.

Es zeigen:

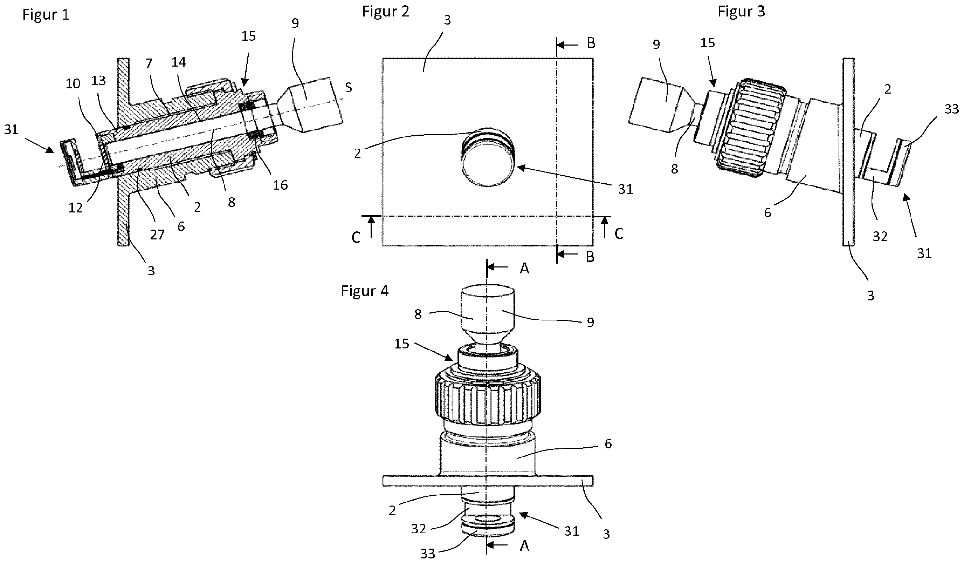

- Figur 1: eine Querschnittsansicht, einer ersten bevorzugten Ausführungsform einer Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 4 dargestellten Schnittebene AA verläuft,
- Figur 2: eine Aufsicht auf die erste bevorzugte Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, schräg von oben, jedoch von der Innenseite des Behälters zur Aufnahme von fluiden Medien aus gesehen,
- Figur 3: eine Querschnittsansicht, der ersten bevorzugten Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 2 dargestellten Schnittebene BB verläuft,
- Figur 4: eine Querschnittsansicht, der ersten bevorzugten Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 2 dargestellten Schnittebene CC verläuft,

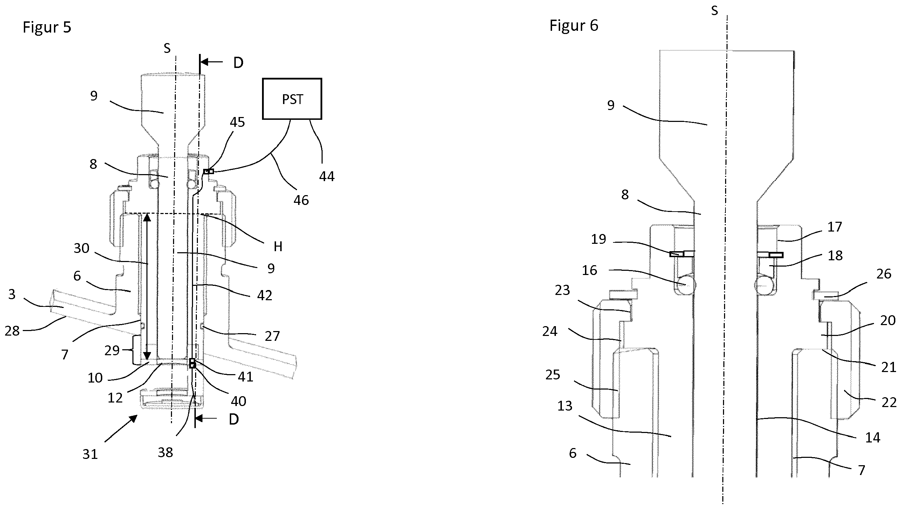

- Figur 5: eine weitere Querschnittsansicht, der ersten bevorzugten Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 4 dargestellten Schnittebene AA leicht zu dieser geneigt verläuft,
- Figur 6: einen Ausschnitt aus der Darstellung der Figur 5, deren oberen Bereich erfassend,

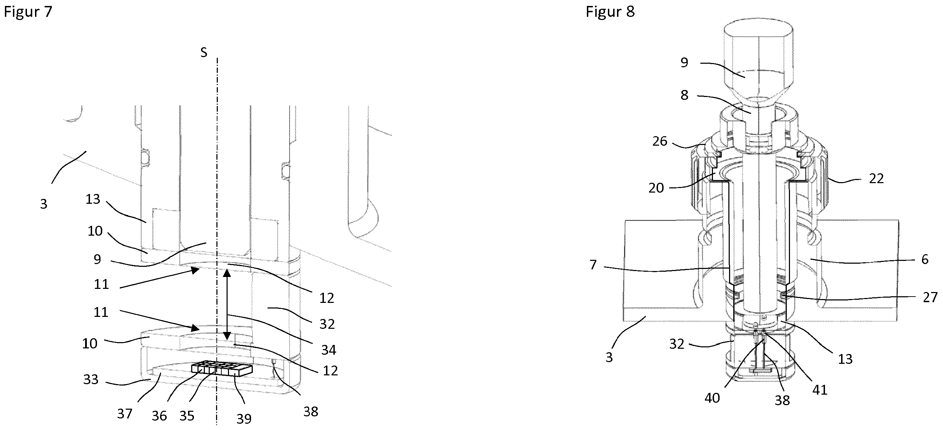

- Figur 7: einen Ausschnitt aus der Darstellung der Figur 5, deren unteren Bereich erfassend, jedoch geringfügig gedreht und geneigt gegenüber der Darstellung aus Figur 5,
- Figur 8: eine perspektivische Schnittdarstellung entlang der Schnittebene DD aus Figur der ersten bevorzugten Ausführungsform einer Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, bei welcher diese transparent dargestellt ist und nur deren Umrisskanten gezeigt sind,

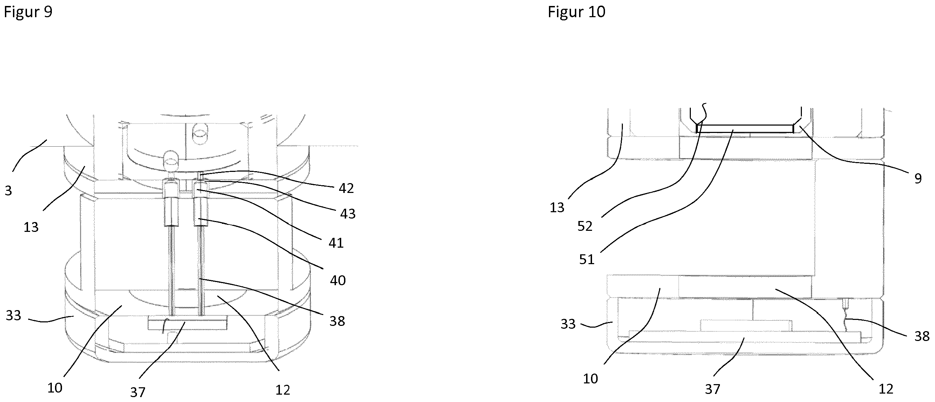

- Figur 9: einen Ausschnitt aus der Darstellung der Figur 8, welcher einen unteren Teil der Figur 8 erfasst,
- Figur 10: einen Ausschnitt analog zu der Darstellung der Figur 5, jedoch eine weitere Ausführungsform darstellend, bei welcher ein Sensor zur Erfassung elektromagnetischer Strahlung in der Vorrichtung zur Halterung einer bildgebenden Einrichtung angeordnet ist, deren unteren Bereich erfassend,

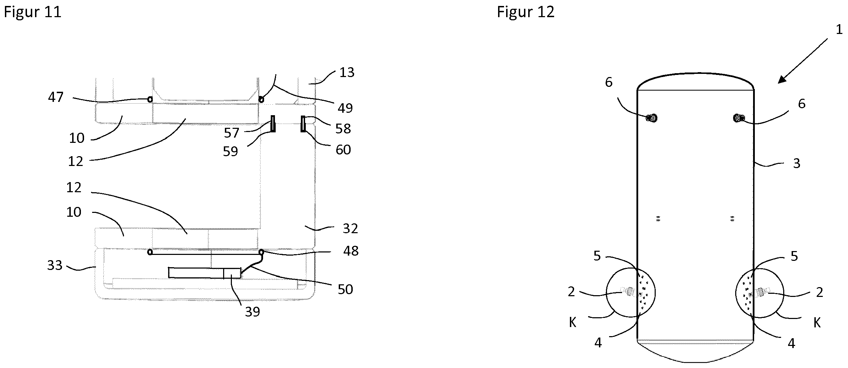

- Figur 11: einen Ausschnitt analog zu der Darstellung der Figur 5, jedoch eine nochmals weitere Ausführungsform darstellend, bei welcher ein induktiver Koppler in einem Halterungskörper der Vorrichtung zur Halterung einer bildgebenden Einrichtung und ein induktiver Koppler in einer Beleuchtungsvorrichtung der Vorrichtung zur Halterung einer bildgebenden Einrichtung angeordnet ist, deren unteren Bereich erfassend,
- Figur 12: eine seitliche Ansicht eines Bioreaktors mit einer Vorrichtung zur Halterung einer bildgebenden Einrichtung, bei welcher der Behälter zur Aufnahme von fluiden Medien, die biologisches Material enthalten teilweise aufgebrochen dargestellt ist,

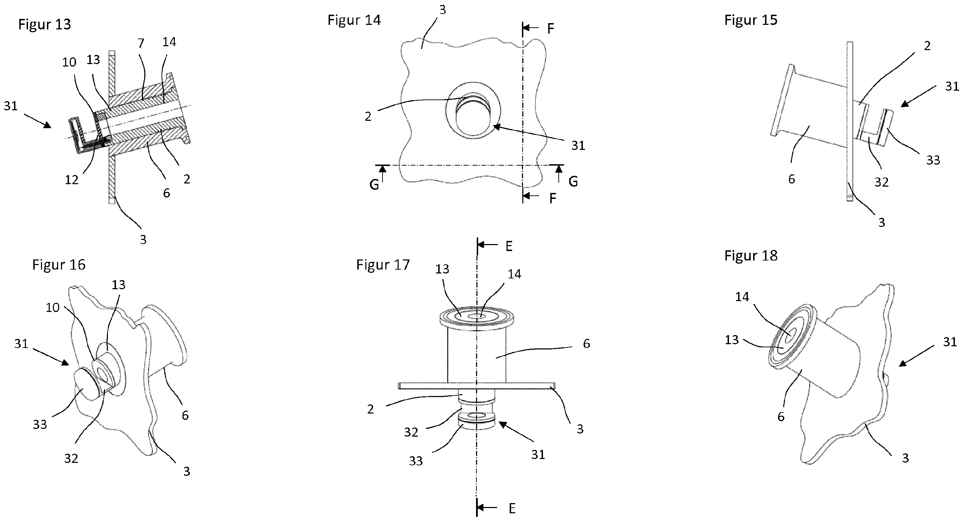

- Figur 13: eine Querschnittsansicht, einer weiteren bevorzugten Ausführungsform einer Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 17 dargestellten Schnittebene EE verläuft, und der Bioreaktor ein Single-Use-Bioreaktor ist,
- Figur 14: eine Aufsicht auf die weitere bevorzugte Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, schräg von oben, jedoch von der Innenseite des Behälters zur Aufnahme von fluiden Medien aus gesehen,
- Figur 15: eine Querschnittsansicht, der ersten bevorzugten Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 14 dargestellten Schnittebene FF verläuft,
- Figur 16: eine weitere Aufsicht auf die weitere bevorzugte Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, schräg von seitlich oben, jedoch von der Innenseite des Behälters zur Aufnahme von fluiden Medien aus gesehen,
- Figur 17: eine Querschnittsansicht, der weiteren bevorzugten Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, wobei die Schnittebene entlang der in Figur 14 dargestellten Schnittebene GG verläuft,
- Figur 18: eine weitere Aufsicht auf die weitere bevorzugte Ausführungsform der Vorrichtung zur Halterung einer bilderfassenden Einrichtung, welche in einer Durchgangsöffnung eines Behälters zur Aufnahme von fluiden Medien, die biologisches Material enthalten, der jedoch nur zum Teil dargestellt ist, angeordnet ist, schräg von seitlich schräg von unten, jedoch von der Außenseite des Behälters zur Aufnahme von fluiden Medien aus gesehen,

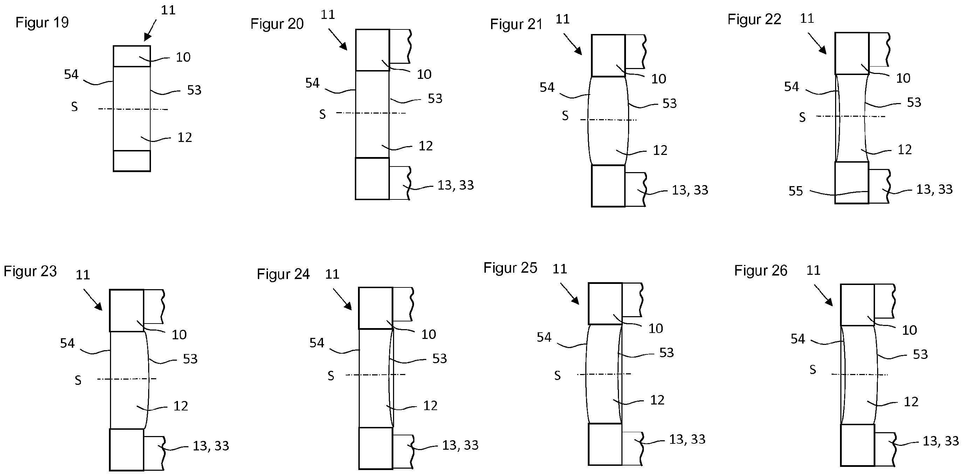

- Figur 19: eine entlang der Symmetrieachse S verlaufende schematische Schnittdarstellung eines hier beschriebenen Fensters zur Erläuterung von dessen Herstellungsverfahren,
- Figur 20: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem planparallelen transparenten Element, wobei die Symmetrieachse S lediglich beispielhaft für ein Fenster in Figur 9 dargestellt ist,
- Figur 21: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem bikonvexen transparenten Element,
- Figur 22: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem bikonkaven transparenten Element,
- Figur 23: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem plankonvexen transparenten Element,
- Figur 24: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem plankonkaven transparenten Element,
- Figur 25: eine entlang der Symmetrieachse S eines Fensters der Vorrichtung zur Halterung einer bilderfassenden Einrichtung verlaufende schematische Schnittdarstellung mit einem Teil des Halterungskörpers der Vorrichtung zur Halterung einer bilderfassenden Einrichtung und einem konvexkonkaven transparenten Element,
- Figur 26: eine entlang der Symmetrieachse S eines Fensters der Vorrichtung zur Halterung einer bilderfassenden Einrichtung verlaufende schematische Schnittdarstellung mit einem Teil des Halterungskörpers Vorrichtung zur Halterung einer bilderfassenden Einrichtung und einem konkavkonvexen transparenten Element,

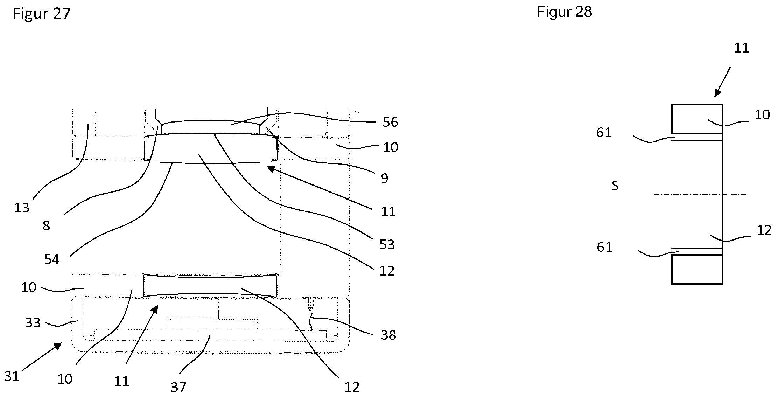

- Figur 27: einen Ausschnitt analog zu der Darstellung der Figur 5, jedoch eine weitere Ausführungsform darstellend, bei welcher ein Fenster einen Teil einer diesem zugeordneten mikroskopischen Einrichtung, insbesondere einer Mikroskopsonde bildet
- Figur 28: eine entlang der Symmetrieachse S verlaufende schematische Schnittdarstellung eines hier beschriebenen Fensters, bei welchem dessen transparentes Element Quarzglas umfasst, zur Erläuterung von dessen Herstellungsverfahren,

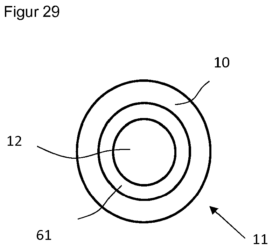

- Figur 29: eine Aufsicht auf das in Figur 23 dargestellte Fester, bei welchem dessen transparentes Element Quarzglas umfasst.

### Detaillierte Beschreibung bevorzugter Ausführungsformen

Bei der nachfolgenden detaillierten Beschreibung bevorzugter Ausführungsformen sind gleiche oder gleich wirkende Bestandteile in den Figuren jeweils mit den selben Bezugszeichen versehen.

Die Figuren sind jedoch um der Klarheit willen nicht maßstabsgerecht dargestellt.

Um der Kürze Willen wird nachfolgend der Behälter zur Aufnahme von fluiden Medien, die biologisches Material enthalten, auch als Behälter zur Aufnahme von fluiden Medien oder noch stärker abgekürzt auch nur als Behälter bezeichnet.

Soweit im Rahmen der vorliegenden Offenbarung der Begriff der fluiden Medien verwendet wird, trägt dieses der Tatsache Rechnung, dass in einem Bioreaktor mehr als ein fluider Bestandteil vorhanden sein kann, beispielsweise ein Trägerfluid, in welchem sich das biologische Material oder eine Vorstufe des biologischen Materials befinden kann, in welchem aber auch fluide Bestandteile des biologischen Materials selbst oder weitere Nährlösung vorhanden sein können. Soweit sich jedoch nur ein Trägerfluid und kein weiterer fluider Bestandteil im Bioreaktor befindet, umfasst der Begriff der fluiden Medien auch dieses Trägerfluid im Singular, ohne dass weitere fluide Bestandteile zusätzlich vorhanden sein müssen.

Das biologische Material umfasst im Rahmen der vorliegenden Offenbarung generell pro- und eukaryotische Zellkulturen, wie Mammalia-zellen, photo-, hetero- und mixotrophen Mikroorganismen und liegt beispielsweise in Form von Mikroalgen, beispielsweise Blaualgen oder Cyanobakterien vor und umfasst insbesondere auch durch Mutagenese veränderten photo- oder mixotrophe Mikroorganismen, insbesondere auch Hefen und Bakterien.

Nachfolgend wir zunächst auf Figur 12 Bezug genommen, welche einem im Ganzen mit dem Bezugszeichen 1 versehenen Multi-Use-Bioreaktor darstellt, an welchem eine ebenfalls im Ganzen mit dem Bezugszeichen 2 bezeichnete Vorrichtung zur Halterung einer bilderfassenden Einrichtung angeordnet ist.

Der Bioreaktor 1 umfasst einen Behälter 3 zur Aufnahme fluider Medien, welche biologisches Material enthalten, wobei sowohl das fluide Medium 4 oder die fluiden Medien 4 als auch das punktförmig dargestellte biologische Material 5 innerhalb des Kreises K, welcher in dessen Inneren eine aufgebrochene Querschnittsdarstellung des Behälter 3 zeigt, zu erkennen sind.

Generell kann der Behälter 3 der Behälter eines Multi-Use-Bioreaktors 1 zur Mehrfachanwendung sein, wie dieser beispielhaft in Figur 12 dargestellt ist, oder auch eines Single-Use-Bioreaktor für Einmalanwendungen, wie dieser beispielhaft in den Figuren 13 bis 18 dargestellt ist.

Vorteilhaft umfasst der Behälter 3 bei einem Multi-Use-Bioreaktor Edelstahl oder besteht dieser Behälter 3 aus Edelstahl. Auch die nachfolgend noch detaillierter beschriebene Vorrichtung 8 zur Halterung einer bilderfassenden Einrichtung 9 kann Edelstahl umfassen oder es können zumindest eines oder mehrere von deren Bestandteilen sowie beispielsweise der in Figur 7 zu erkennende der Grundkörper 10 der hier offenbarten Fenster 11 aus Edelstahl bestehen oder dieses umfassen.

Verwendbar sind hierbei alle Edelstähle, insbesondere auch austenitische und ferritische Edelstähle, bevorzugt jedoch nur soweit diese bei der Durchführung der Erfindung rostfrei bleiben.

Der Edelstahl kann bevorzugt auch 316L Pharmastahl umfassen, oder vollständig aus diesem bestehen.

Ferner sind prinzipiell auch Titan sowie Monell-Legierung mit hohem Kupferanteil verwendbar, wobei das Material bei Verwendung für die Beleuchtungsvorrichtung insbesondere für deren Gehäusekörper auch emailliert sein kann.

Bei Single-Use-Reaktoren 1 kann der Behälter 3 zur Aufnahme von fluiden Medien, die biologisches Material enthalten, einen Kunststoff, insbesondere einen sterilisierbaren Kunststoff umfassen oder aus einem Kunststoff, insbesondere einem sterilisierbaren Kunststoff bestehen. In diesem Fall hat dann der Behälter 3 nicht die in Figur 12 dargestellte Form sondern kann auch als Beutel ausgebildet sein.

Ferner können an dem Behälter 3 mehr als ein Port 6, welche jeweils auch als Durchführung 6 bezeichnet werden, angeordnet sein.

Bevorzugt umfassen diese Durchführungen 6, insbesondere auch bei der in Figur 12 dargestellten Ausführungsform einen Standardport, wie beispielsweise einen Ingold-Port, einen Broadly-James- Port, einen B.-Braun-Sicherheitsport oder einen einem anderen Standard entsprechenden Port.

Bei Einwegreaktoren oder Single-Use-Reaktoren kann die Durchführung 6 auch einen Tri-Clamp-, einen Sanitary-Clamp-Port oder einen herstellerspezifisch ausgebildeten Port umfassen, wie dieses beispielhaft den Figuren 13 bis 18 zu entnehmen ist.

Die Durchführungen 6 weisen jeweils eine Durchgangsöffnung 7 definierten Durchmessers auf, welche in typischer Weise das Innere eines Bioreaktors 1 mit dessen Äußerem verbindet oder den Zugang zum Behälter 3 eröffnet.

Besonders gut sind diese Durchgangsöffnungen 7 der Ports 6 auch jeweils in den Figuren 1, 5 und 6 zu erkennen.

Innerhalb zumindest einer Durchgangsöffnung 7 ist eine Vorrichtung 2 zur Halterung einer bilderfassenden Einrichtung 9, insbesondere einer mikroskopischen Einrichtung, angeordnet, wobei die Vorrichtung 2 zur Halterung einer bilderfassenden Einrichtung 9 zumindest teilweise in der Durchgangsöffnung 7 der Durchführung 6 gehalten ist, denn im Rahmen der vorliegenden Offenbarung offenbart dies, dass die Vorrichtung 2 bereichsweise sowohl in das Innere des Behälters 3 als auch über die Durchgangsöffnung 7 der Durchführung 6 hinaus zum Äußeren des Behälters 3 hinausragen kann.

Der Bioreaktor 1 kann auch über mehrere Durchführungen 6 verfügen, in welchen jeweils eine Vorrichtung 2 zur Halterung einer bilderfassenden Einrichtung 9, angeordnet ist.

Dabei können jeweils die gleiche Art von bilderfassender Einrichtung 9 oder können auch verschiedene Ausführungsformen der bilderfassenden Einrichtung 9 in der Vorrichtung 2 angeordnet sein.

Eine erste bevorzugte Ausführungsform umfasst beispielsweise eine dem Fachmann an sich bekannte Mikroskopsonde 8, welche eine im wesentlichen zylindrische Gestalt aufweist und lösbar aber dennoch fest an der Vorrichtung 2 gehalten ist.

Nachfolgend wird unter Bezugnahme auf die Figuren 1 bis 4 die Vorrichtung 2 zur Halterung einer bilderfassenden Einrichtung 8 detaillierter beschrieben.

Wie besonders gut aus Figur 1 zu erkennen ist, umfasst die Vorrichtung 2 einen Halterungskörper 13, welcher zylindersymmetrisch, insbesondere säulenförmig und vorzugsweise einstückig ausgebildet ist.

Der Halterungskörper 13 weist eine vorzugsweise zylindrische Durchgangsöffnung 14 auf, welche auf der dem Inneren des Bioreaktors 1 zugeordneten Seite mittels einem Fensters 11 fluiddicht, insbesondere hermetisch dicht abgeschlossen ist.

Wie beispielsweise auch in Figur 7 gut zu erkennen ist, umfasst das Fenster 11 ein transparentes Element 12, welches in einem Grundkörper 10 fluiddicht, insbesondere hermetisch dicht gehalten ist.

Das transparente Element 12 ist für elektromagnetische Strahlung transparent, denn dieses umfasst Glas oder besteht aus Glas, wobei das Glas bevorzugt Quarzglas oder Borosilikatglas enthält oder aus diesem besteht.

Der Halterungskörper 13 bildet zusammen mit dem Fenster 11 eine Halterung 15 für die bilderfassende Einrichtung 9, in welcher die bilderfassende Einrichtung 9 lösbar befestigbar gehalten ist.

Hierzu kann der Halterungskörper 13, wie beispielsweise auch Figur 6 zu entnehmen ist, vorzugsweise mittels eines Reibelements 16, vorzugsweise einem O-Ring eine reibschlüssige Verbindung zu der bilderfassenden Einrichtung 9 eingehen.

Das Reibelement 16 ist in einer zylindrischen Ausnehmung 17 des Halterungskörpers 13 durch ein im Wesentlichen ringförmiges Druckelement 18 gehalten, welches in axialer Richtung des Halterungskörpers 13 eine definiert einstellbare Kraft auf das Reibelement ausübt.

Durch die dabei entstehende definierte Deformation des Reibelements 16 kann die bilderfassende Einrichtung 9 mit einer daraus resultierenden definierten Kraft innerhalb der Durchgansöffnung 14 gehalten werden, welche betriebssicher deren Lage sicherstellt aber dennoch eine zügige manuelle Entnahme aus der Durchgangsöffnung 14 oder auch ein zügiges manuelles Einbringen in die Durchgangsöffnung 14 ermöglicht.

Mittels eines Sprengrings 19 ist das ringförmige Druckelement 18 in seiner Lage definiert fixiert.
Der Halterungskörper 13 selbst liegt mit einem seitlichen, sich in radialer Richtung erstreckenden Absatz 20 an einem oberen Flansch 21 der Durchführung 6 formschlüssig an.

Mittels einer Hutmutter 22 mit einer zylindrischen Durchgangsöffnung 23, welche den sich in radialer Richtung erstreckenden Absatz 20 übergreift, ist der Halterungskörper 13 lösbar, jedoch ortsfest an dem Port 16 gehalten.

Hierzu weist sowohl die Hutmutter 22 ein Gewinde 24 als auch der Halterungskörper 13 ein Gegengewinde 25 auf.

Mittels eines Sprengrings 26 ist die Hutmutter 22 drehbar jedoch axial nur mit geringem Spiel unverlierbar am Halterungskörper 13 gehalten.

Durch Verdrehen der Hutmutter 2 kann die Vorrichtung 2 zur Halterung einer bilderfassenden Einrichtung zügig an dem jeweiligen Port 6 betriebssicher montiert oder auch von diesem gelöst werden.

Mittels eines Dichtelements 27, beispielsweise eines O-Rings, ist der Halterungskörper 13 fluiddicht und vorzugsweise hermetisch dichtend gegenüber der Durchgangsöffnung 7 des Behälters 3 form- und reibschlüssig in der Durchgangsöffnung 7 gehalten.

Hierbei definiert die Symmetrieachse S des Halterungskörpers 13, welche beispielsweise auch in den Figuren 1 und 5 zu erkennen ist, die axiale oder Längsrichtung, auf welche jeweils im Rahmen der vorliegenden Offenbarung Bezug genommen wird.
Bei einer in die Durchgangsöffnung 2 eingesetzten und vorzugsweise wie vorstehend beschrieben fixierten Vorrichtung 2 zur Halterung einer bilderfassenden Einrichtung 9 wird der axiale Abstand 29 des Fensters 11 zur Innenwand 28 des Bioreaktors 1 durch den axialen Abstand 30 des Fensters 11 zum seitlichen Absatz 20 definiert.

Der axiale Abstand des Fensters 11 zum seitlichen Absatz 20 wird dabei von der Unterseite des seitlichen, sich in radialer Richtung erstreckender Absatzes 20 ausgehend, welches durch eine Hilfslinie H angedeutet ist, bis zur Oberseite des Grundkörpers 10 des Fensters 11 gemessen, wie dieses beispielsweise in Figur 5 zu erkennen ist.

Der axiale Abstand 29 des Fensters 11 zur Innenwand 28 des Bioreaktors 1 ist hierbei der größte Abstand zwischen der Unterseite des Grundkörpers 11 zur Innenwand 28 des Bioreaktors 1, somit zur Innenwand 28 des Behälters 3 des Bioreaktors 1.

Bevorzugt ist hierbei das transparente Element 12 des Fensters 11 bei in die Durchgangsöffnung 7 eingesetztem Halterungskörper 13, insbesondere bei Anlage des seitlichen Absatzes 20 an einem Standardport 6, wie beispielsweise in Figur 5 gezeigt, innerhalb des Bioreaktors 1 angeordnet.

Ein Satz von Halterungskörpern 13 mit verschiedenem axialem Abstand 30 des transparenten Elements 12 des Fensters 11 zum seitlichen Absatz 20 kann hierbei eine wählbare axiale Positionierung des transparenten Elements 12 des Fensters innerhalb des Bioreaktors 1 ermöglichen.

Hierdurch können bei Verwendung mehrerer Vorrichtungen 2 mit jeweils verschiedenem axialen Abstand 30 des Fensters 11 zum seitlichen Absatz 20 jeweils verschiedene Orte des Bioreaktors 1 erfasst werden. Somit kann durch dieses Vorgehen ein Bioreaktor 1 bei Verwendung mehrere Vorrichtungen 2 in dessen lokalen Abläufen jeweils erheblich besser erfasst werden.

Wie beispielsweise in den Figuren 1 bis 4 zu erkennen ist, kann die Vorrichtung 2 zur Halterung einer bilderfassenden Einrichtung eine Beleuchtungsvorrichtung umfassen, welche insgesamt mit dem Bezugszeichen 31 versehen ist und entweder dauerhaft oder lösbar am Halterungskörper 13 anbringbar ist.

Diese Beleuchtungsvorrichtung 31 wird nachfolgend unter Bezugnahme auf die Figuren 7, 8 und 9 detaillierter beschrieben.

Die Beleuchtungsvorrichtung 31 umfasst einem Sockelabschnitt 32, an welchem ein Fußteil 33 gehalten ist.

Der Fußteil 33 bildet einen im Wesentlichen topfförmigen unteren Abschnitt und somit ein Gehäuse aus, an welchem ein weiteres Fenster 11 mit einem weiteren Grundkörper 10 und einem weiteren transparentem Element 12 hermetisch dicht angeordnet ist.

Das transparente Element 12 des Fensters 11 des Fußteils 33 erstreckt sich im Wesentlichen parallel zu dem transparenten Element 12 des am Halterungsteil angeordneten Fensters 11.

Im montierten Zustand liegt das an dem Halterungskörper 13 angebrachte Fenster 11 dem Fenster 11 des Fußteils 33 oder Gehäuses 33 gegenüber.

Somit bilden die Vorrichtung 2 zur Halterung einer bilderfassenden Einrichtung 9 und deren Beleuchtungsvorrichtung 31 im montierten Zustand innerhalb des Behälters 3 eine Kammer mit einem definierten, insbesondere zu zumindest zwei Seiten senkrecht zur Symmetrieachse S des Halterungsköpers 13 hin offenem Volumen aus.

Bevorzugt weisen das transparente Element 12 des Fensters 11 der Beleuchtungsvorrichtung 31 und das transparente Element 12 des Fensters 11, welches am Halterungskörper 13 der Vorrichtung 2 zur Halterung einer bilderfassenden Einrichtung 9 angeordnet ist, einen definierten Abstand, welcher in Figur 7 das Bezugszeichen 34 trägt, zueinander auf.

Hierdurch wird zwischen den beiden Fenstern 11, 11 ein definiertes Messvolumen bereitgestellt, welches beispielsweise eine Trübungskammer bereitstellt, falls definiert Licht in diese eingestrahlt und entsprechend Messungen mittels eines Sensors 51 vorgenommen werden, welche nachfolgend noch detaillierter beschrieben werden.

Bevorzug kann hierfür oder aber auch für die bildgebende Erfassung und/oder Aufzeichnung mittels einer Mikroskopsonde 8 die Beleuchtungsvorrichtung 31 eine LED 35 oder einen Array 36 von LEDs 35 umfasst, die in dem Gehäuse 33 oder Fußteil 33 der Beleuchtungsvorrichtung 31 angeordnet und an einer gedruckten Schaltungsplatine 37, mit welcher zumindest ein Mehrfachleiter 38 verbunden ist, gehalten sind.

Von den LEDs 35 ist um der Klarheit Willen lediglich eine innerhalb des Arrays 36 von LEDs mit einem Bezugszeichen versehen, jedoch liegt auch die Verwendung einer einzigen LED 35 im Rahmen dieser Offenbarung.

Zusätzlich befindet sich ein Kontroller 40 auf der gedruckten Schaltungsplatine 37 und ist mittels dieser mit dem Mehrfachleiter 38 verbunden.

Die Beleuchtungsvorrichtung 31 weist an deren Gehäuse zumindest einen elektrischen Verbinder 40 auf, mit welchem der Mehrfachleiter 38 verbunden ist.

Auch die Vorrichtung 2 zur Halterung einer bilderfassenden Einrichtung 9 umfasst an deren Halterungskörper 13 zumindest einen elektrischen Verbinder 41, wie dieses beispielsweise auch den Figuren 8 und 9 zu entnehmen ist, welcher durch den Mehrfachleiter 42 mit einer externen Steuerungseinrichtung verbunden ist.

Die elektrischen Verbinder 40, 41 sind miteinander fügekompatibel und jeweils hermetisch abgedichtet.

Bei der in den Figuren dargestellten steckbaren Ausführung der elektrischen Verbinder 40, 41 wird das Gehäuse des elektrischen Verbinders 40, 41 jeweils über eine elastomere Komponente, bei dem Verbinder 40 gegenüber dem Fußteil 33 und bei dem Verbinder gegenüber dem Halterungskörper 13 gedichtet, wie dieses in Figur 9 für den Verbinder 41 gezeigt ist. Als elastomere Komponente sind hierbei Werkstoffe wie EPDM, NBR, FKM verwendbar.

Die Mehrfachleiter 39, 42 können sowohl Signale eines Kommunikationsbusses als auch jeweilige Versorgungsleitungen, insbesondere für den Kontroller 38 als auch die LEDs 35 umfassen.

Ein bevorzugter Kommunikationsbus umfasst beispielsweise den Datenaustausch über OPC XML und umfasst JAVAprogrammierbare Clients. Ein derartiger Kommunikationsbus ist mit dessen Clients beispielsweise beschrieben in SIMOTION, Beschreibung und Beispiel zum Datenaustausch über OPC XML Schnittstelle, Version 1.0 Ausgabe 07/2007 publiziert durch die Siemens AG.

Alternativ oder zusätzlich können die Mehrfachleiter 38, 42 auch analoge Signale führen und die LEDs 35 des Arrays 36 direkt durch eine zugeordnete, vorzugsweise externe elektronische Einrichtung angesteuert, insbesondere auch stroboskopisch angesteuert werden, wobei die Prozessteuerungseinrichtung (PST) 44 in Figur 5 lediglich schematisch dargestellt ist, jedoch hierbei zu erkennen ist, dass die Prozesssteuereinrichtung 44 über einen nochmals weiteren Mehrfachleiter 46 mittels weiterer Verbinder, wie beispielhaft durch den Verbinder 45 gezeigt ist, mit dem Mehrfachleiter 42 verbunden ist.

Wie aus Figur 5 zu erkennen ist, verfügt der Halterungskörper 13 entsprechend an dessen oberem Abschnitt über diesen weiteren Verbinder 45, an welchen der Mehrfachleiter 42 angeschlossen ist.

Zur internen Führung der Mehrfachleiter 38, 42 und 49 können jeweils Durchgangsbohrungen oder entsprechende Sacklochbohrungen in dem Halterungskörper 13, dem Sockelabschnitt 32 und dem jeweiligen Grundkörper 10 eines Fensters 11 angebracht sein, welche diese oder diesen aufnehmen, und deren Anbringung für den Fachmann jeweils ersichtlich ist, welche jedoch lediglich um der Klarheit Willen nicht in den Figuren dargestellt sind.

Ferner kann, wie beispielhaft in Figur 11 dargestellt ist, die Vorrichtung 2 zur Halterung einer bilderfassenden Einrichtung 9 an deren Halterungskörper 13 zumindest einen induktiven Koppler 47 und die Beleuchtungsvorrichtung 2 an deren Gehäuse 33 oder Fußteil 33 zumindest einen induktiven Koppler 48 aufweisen, welche miteinander im montierten Zustand induktiv koppelbar und jeweils, insbesondere gegenüber dem oder den fluiden Medien 4 hermetisch abgedichtet sind.

Mittels dieser Koppler 47, 48 kann vom Halterungskörper 13 ausgehend sowohl elektrische Energie zur Versorgung des Kontrollers 39 als auch der LEDs 35 des Arrays von LEDs eingekoppelt und in einer elektrischen Speichereinrichtung des Kontrollers 39 in für die hier beschriebenen Vorgänge ausreichenden Menge eingespeist werden.

Hierzu kann der induktive Koppler 47 über einen Mehrfachleiter 49 mit dem weiteren Verbinder 45 und der induktive Koppler 48 über einen weiteren Mehrfachleiter 50 mit dem Kontroller 39 verbunden sein.

Ferner können auch zeitlich oder Frequenz-modulierte Signale durch die Koppler 47, 48 bidirektional übertragen werden, sodass auch bei dieser Ausführungsform eine entsprechende Steuerung durch die Prozesssteuerungseinrichtung 44 vorgenommen werden kann.

Um sowohl die elektrische Energie als auch die zeitlich oder Frequenz-modulierten Signale störungssicher übertragen zu können, umfassen die induktiven Koppler 47, 48 jeweils eine Spule mit einer Windung oder mit mehreren Windungen, von welchen jedoch in der Schnittdarstellung der Figur 11 jeweils nur eine einzige Windung beispielhaft gezeigt ist.

Ferner können auch definiert angesteuert, insbesondere wie vorstehend beschrieben jeweils nur einzelne Lichtpulse von den LEDs 35 der Beleuchtungsvorrichtung abgegeben werden, insbesondere unter Ansteuerung der dieser zugeordneten elektronischen Einrichtung, vorliegend der Prozesssteuerungseinrichtung (PST) 44. Synchron hierzu kann beispielsweise eine Mikroskopsonde 8 mit einer bildaufzeichnenden Einrichtung während der Abgabe jeweils eines der bei der stroboskopischen Beleuchtung abgegebenen Lichtpulse oder Pulse elektromagnetischer Strahlung jeweils ein Bild erfassen, wodurch beispielsweise durch die Bewegung des fluiden Mediums 4 oder der fluiden Medien 4 sowie des biologischen Materials 5 verursachte Unschärfen bei der Bildaufzeichnung erheblich vermindert werden können.

Eine weiter bevorzuge Ausführungsform ist in Figur 10 dargestellt, bei welcher die bilderfassende Einrichtung 9 einen Sensor 51 zum Messen der Wellenlänge und/oder Intensität von elektromagnetischer Strahlung anstelle der Mikroskopsonde 8 oder in diese integriert umfasst.

Der Sensor 51 zum Messen der Wellenlänge und/oder Intensität von elektromagnetischer Strahlung kann genutzt werden, um einerseits die Intensität und/oder Wellenlänge der von den LEDs 35 emittierten Strahlung zu messen. Da diese einem Alterungsprozess unterliegen und/oder deren Emissionsspektrum z.B. von der Umgebungstemperatur abhängen kann, ist deren Emission im Betriebszustand mit Hilfe des Sensors 51 steuerbar. Der zumindest eine Sensor 51 kann aber auch dazu genutzt werden, um den Zustand des biologischen Materials 5 im Bioreaktor 1 zu kontrollieren und/oder zu charakterisieren. Durch Streuung oder Rückstreuung des im Betriebszustand beleuchteten biologischen Materials 5 kann beispielsweise die Wellenlänge der vom Sensor empfangenen elektromagnetischen Strahlung Informationen über das biologische Material 5 liefern. Wird das biologische Material 5, beispielsweise bestimmte Algen beispielsweise mit weißem Licht für insbesondere deren Wachstum beleuchtet, streuen diese beispielsweise grünes Licht zurück. Die Intensität des grünen Lichts kann von der Konzentration der Algen und/oder deren Wachstumszustand abhängig sein. Diese Informationen können genutzt werden, um die Prozesse im Bioreaktor 1 zu steuern, beispielsweise Nährstoffzufuhr, Temperatur und weitere. Ebenso möglich ist selbstverständlich auch das Messen von Fluoreszenz und weiteren optischen Eigenschaften.

In Figur 10 ist der Sensor 51 als eigenständiges Bauelement dargestellt und es umfasst im Sinne der vorliegenden Offenbarung dann die bilderfassende Einrichtung 9 quasi nur einen Bildpunkt.

Wenn jedoch die Vorrichtung 2 zur Halterung einer bilderfassenden Einrichtung 9 zumindest einen Sensor, insbesondere innerhalb einer Mikroskopsonde 8 oder dieser Mikroskopsonde 8 zugeordnet eine bildaufzeichnende Einrichtung aufweist, und im Betriebszustand die Strahlungsintensität und/oder Wellenlänge der elektromagnetischen Strahlung im Innern des Bioreaktors 1 insbesondere im Inneren des Behälters 3 mit dieser bilderfassenden Einrichtung aufgenommen wird, kann hierbei die Strahlungsintensität und/oder die Wellenlänge ortsaufgelöst gemessen werden. Beispielhaft sind zur Messung der Wellenlänge nachfolgend auch Beschichtungen auf dem transparenten Element 12 beschrieben. Ferner können innerhalb einer Mikroskopsonde 8 angeordnete Filter sowohl optischer als auch elektronischer Art beispielsweise in der bildaufzeichnenden Einrichtung hierzu verwendet werden.

Mit einem derartigen Sensor 51 kann beispielsweise auch eine Trübungsmessung vorgenommen werden, indem die durch das Fenster 11 der Beleuchtungsvorrichtung 31 austretende elektromagnetische Strahlung nach Durchtritt durch das an der Halterungseinrichtung, beziehungsweise Halterungskörper 13 angebrachte Fenster 11 durch diesen Sensor 51 erfasst wird. Auch eine derartige Messung kann unter Steuerung der Prozesssteuerungseinrichtung (PST) 44 durchgeführt werden und ist hierzu der Sensor 51 mittels eines Mehrfachleiters 52 mit der Prozesssteuerungseinrichtung (PST) 44, beispielsweise ähnlich wie in Figur 5 für den Kontroller 39 gezeigt, verbunden.

Eine Prozesskontrolle mittels eines in-situ Mikroskops ermöglicht hierbei die Erfassung quantitativer und morphologischer Informationen über die jeweiligen Zellen des biologischen Materials 5. Daraus abgeleitet auch direkt die Optimierung der Einflussgrößen für eine optimierte oder zumindest verbesserte Ausbeute. Diese sind beispielsweise auch die Begasungsrate, die Begasungszusammensetzung, Temperatur, pH, rX, Konzentrationen der gelösten Gase, Durchmischung/Rührerdrehzahl, Feedrate, Feedzusammensetzung, Kultivierungszeit, aktivierende oder inhibierende Faktoren und können auch noch weitere Einflussgrößen umfassen.

Bei den vorliegend bevorzugten Ausführungsformen bildet ein Fenster 11 oder bilden beide Fenster 11 jeweils eine Einglasung für ein transparentes Element 12, vorzugsweise eine GTMS-Druckeinglasung aus, wie dieses nachfolgend unter Bezugnahme auf Figur 19 beschrieben wird.

Figur 19 zeigt eine entlang der Symmetrieachse S verlaufende schematische Schnittdarstellung eines hier beschriebenen Fensters 11 zur Erläuterung von dessen Herstellungsverfahren.

Das in Figur 19 dargestellte Fenster 11 umfasst einen aus Stahl bestehenden, ring- oder zylinderförmigen Grundkörper 10, welcher das transparente Element 12 seitlich umgibt und auf dieses eine Druckkraft ausübt, die eine dauerhaft hermetische und für die Zwecke der vorliegenden Erfindung ausreichend druck- sowie temperaturfeste Verbindung zwischen dem transparenten Element 12 und dem Grundkörper 10 sicherstellt.

Bei seiner Herstellung wird das aus Glas bestehende oder Glas umfassende transparente Element vorzugsweise in etwa bereits seiner endgültigen Form entsprechend innerhalb des Grundkörpers 10 angeordnet und mit diesem zusammen so lange erwärmt, bis das Glas des transparenten Elements 12 dessen Glastemperatur Tg oder dessen Halbkugeltemperatur überschritten hat und beginnt, am Grundkörper 10 anzuschmelzen.

Nach erfolgtem Anschmelzen wird dann der Verbund aus transparentem Element 12 und Grundkörper 10 auf Raumtemperatur abgekühlt, wodurch jeweils ein Fenster 11 mit einem im Wesentlichen scheibenförmigen transparentem Element 12 ausgebildet wird.

Da der thermische Ausdehnungskoeffizient des Edelstahls des Grundkörpers 10 größer als der des Glases des transparenten Elements 12 ist, übt dieser, sobald das Glas des transparenten Elements sich zu verfestigen beginnt, eine mit weiter abnehmender Temperatur ansteigende Druckspannung auf das Glas des transparenten Elements 12 aus.

Der Grundkörper 10 hält das transparente Element 12 dann, nach erfolgter Abkühlung, mit dieser quasi eingefrorenen Druckspannung dauerbetriebsfest hermetisch dicht und temperaturstabil.

Eine derartige Druckverglasung wird im Rahmen der vorliegenden Offenbarung auch als Glas-Metall-Verbindung oder Glas-to-Metal-Seal, GTMS, oder GTMS-Druckverglasung bezeichnet.

Bei einer derartigen Glass-to-Metall-Verbindung übt das Metall über den gesamten Betriebstemperaturbereich Druckkräfte auf das Glas aus, insbesondere auch bei Temperaturen bis zumindest 121° C, vorzugsweise sogar 141° Celsius, welche eine Druckspannung zwischen dem Metall und dem Glas ausbilden und dazu beitragen, dass die Glass-Metall-Verbindung dauerhaft betriebssicher sowohl fluidals auch hermetisch dicht ist.

Ferner treten bei derartigen Glas-Metall-Verbindungen in der Regel keine Spalte auf, wie diese bei Verwendung von herkömmlichen Dichtmitteln, beispielsweise von O-Ringen auftreten können und Raum für Kontaminationsherde bieten, welche häufig nur schwer entfernbar sind.

Vorteilhaft ist hierbei ein Unterschied der thermischen Ausdehnungskoeffizienten, welcher die Druckspannung zwischen dem Glas des transparenten Elements 35 und dem Metall des ring- oder zylinderförmiger Grundkörper 36 des Fensters 8 zumindest über den Betriebstemperaturbereich zuverlässig aufrechterhält.

Dieser Unterschied des Ausdehnungskoeffizienten CTE_{M} des Metalls und dem Ausdehnungskoeffizienten des Glases CTE_{G} des transparenten Elements 3 kann beispielsweise kleiner als 80 * 10-⁶ /K, bevorzugt kleiner als 30 * 10⁻⁶ /K oder besonders bevorzugt auch kleiner als 20 * 10⁻⁶ /K betragen. Hierbei soll der thermische Ausdehnungskoeffizient des Metalls CTE_{M} jeweils größer als der thermische Ausdehnungskoeffizient des Glases CTE_{G} sein. In allen Fällen soll dieser Unterschied vorzugsweise jedoch mindestens 1 mal 10⁻⁶ /K betragen.

Quarzglas weist beispielsweise ein CTE_{G} von 0, 6 * 10⁻⁶ /K auf und kann beispielsweise mit Edelstählen mit einem CTE_{M} von 17-18 * 10⁻⁶ /K kombiniert werden.

Der Bioreaktor 1 sowie Vorrichtung zur Halterung einer bilderfassenden Einrichtung 2 ist jeweils einzeln eigenständig oder es sind der Bioreaktor 1 sowie Vorrichtung 2 zur Halterung einer bilderfassenden Einrichtung auch zusammen autoklavierbar. Dies bedeutet, dass insbesondere die Vorrichtung zur Halterung einer bilderfassenden Einrichtung mit deren hier offenbarten Fenstern 11 hermetisch dicht ist und dabei einer Behandlung mit Sattdampf einer Temperatur von 121° C, insbesondere auch von 141° C standhält, ohne dass der Sattdampf oder durch diesen gebildete Fluide in die Vorrichtung 2 eintreten können.

Autoklavierbar wird im Rahmen dieser Offenbarung auch als autoklavierbar im Sinne der DIN EN ISO 14937; EN ISO 17665, welche für Medizinprodukte gültig ist, verstanden.

Überraschend hat es sich gezeigt, dass bei den hier offenbarten Vorrichtungen zur Halterung einer bilderfassenden Einrichtung 3500 Auktoklavierungs-Zyklen bei 2 bar und 134°C möglich waren.

Hierdurch wird auch das dem Fachmann bekannte und vorteilhafte Steaming-in-Place ermöglicht
In diesem dauerbetriebsfest hermetisch dicht und temperaturstabil gehaltenen Zustand kann das Glas des transparenten Elements 12, vorzugsweise nach Prüfung der jeweiligen seitlichen Hauptoberfläche 53, 54 entweder direkt verwendet oder weiteren Oberflächen-bearbeitenden Verfahren, wie beispielsweise Polieren oder formgebenden Schleifen zugeführt werden.

Auf diese Weise kann das jeweilige transparente Element 12 planparallele seitliche Hauptoberflächen 53 und 54 ausbilden oder kann das transparente Element 12 auch plankonvex, plankonkav, bikonvex, bikonkav, konvexkonkav oder konkavkonvex geformt werden, wie dieses den Schnittdarstellungen der Figuren 20 bis 26 zu entnehmen ist.

Bei geringeren optischen Anforderungen an die Oberflächenqualität, insbesondere für zur Messung mit der in Bezug auf Figur 10 beschriebenen Ausführungsform verwendete Strahlengänge, kann das transparente Element 12 auch während des Herstellungsverfahrens in einer entsprechenden Negativ-Form gehalten sein, welche im Wesentlichen bereits dessen finaler Form entspricht.

Die Hauptoberflächen 53, 54 können eine Beschichtung aufweisen, welche wellenlängenselektiv ist und derart ein optisches Bandpass- oder Kantenfilter ausbilden. Mittels eines derartigen optischen Filters können mit der Beleuchtungsvorrichtung 31 definierte Wellenlängen in den Behälter 3 eingestrahlt und identische oder auch von diesen abweichende Wellenlängen gemessen, insbesondere sensorisch mittels des Sensors 51 erfasst werden. Diese Beschichtung kann jeweils nur auf einer oder auch auf beiden Hauptoberflächen 53, 54 vorhanden sein.

Ohne eine derartige, insbesondere ohne jegliche Beschichtung weist das transparente Element 12 zumindest eines Fensters 11 in einem Spektralbereich mit einer Wellenlänge von 250 bis 2000 nm eine Transmission auf, die höher ist als 80%, besonders bevorzugt höher als 90 %.

Wenn das transparente Element 12 des Fensters 8, wie es beispielsweise in den Figuren 28 und 29 gezeigt ist, aus Quarzglas besteht oder Quarzglas umfasst, kann anstelle einer Laserschweißnaht auch ein weiteres Glas 61 oder Glaslot 61, insbesondere bleifreies Glas 61 oder Glaslot 61 verwendet werden, um das Quarzglas hermetisch dicht mit dem ring- oder zylinderförmiger Grundkörper 10 des Fensters 11, insbesondere fluid- und hermetisch dicht zu verbinden. Bevorzugt wird der Grundkörper 10 der Fenster 11 direkt, insbesondere mittels Laser-Schweißen, jeweils hermetisch dicht mit dem Halterungskörper 13 und/oder dem Gehäuse 33 der Beleuchtungsvorrichtung 31 verbunden, sodass hierdurch das Gehäuse 33 hermetisch dicht ausgebildet ist und der Halterungskörper 13 an dessen unterem Ende hermetisch dicht gegenüber dem Inneren des Behälters 3 ebenfalls hermetisch dicht ausgebildet ist.

Beispielhaft ist die hierbei jeweils entstehende Laserschweißnaht lediglich in Figur 22 mit dem Bezugszeichen 55 dargestellt.

Wird nun Licht von einer der LEDs 35 ausgestrahlt, tritt dieses jeweils durch das vor dieser LED 35 angeordnete Fenster 11 und kommt es zu einer definierten Ausleuchtung des sich zwischen den beiden Fenstern 11 befindlichen Volumens.

Eine weitere bevorzugte Ausführungsform ergibt sich, wenn beispielsweise das in Figur 21 dargestellte Fenster zusammen mit einer Mikroskopsonde 8 verwendet wird, wie dieses beispielsweise der Figur 27 zu entnehmen ist, und dabei dieses Fenster 11 einen Teil der diesem zugeordneten mikroskopischen Einrichtung, nämlich der Mikroskopsonde 8 bildet. Hierbei kann beispielsweise bereits die untere Hauptoberfläche 54 oder auch beide Hauptoberflächen 54, 53 strahl- oder wellenformende Wirkung entfalten und lassen sich prinzipiell höhere numerische Aperturen für den bildgebenden Strahlengang erreichen, da dem ersten optische Linsenelement 56 der Mikroskopsonde 8 ein größerer effektiver Winkelbereich für die eintretende elektromagnetische Strahlung zur Verfügung gestellt werden kann.

Hierdurch kann nicht nur die Auflösung der Mikroskopsonde 8 erhöht, sondern auch die insgesamt zur Verfügung gestellte Intensität der elektromagnetischen Strahlung gesteigert werden, wodurch sich bei Verwendung dieses in Figur 21 dargestellten Fensters, insbesondere auch für die in Figur 10 dargestellte Ausführungsform, ein verbesserter Signalzu-Rausch-Abstand für die hierbei erhaltenen und beispielsweise in elektrische Signale gewandelten Messergebnisse ergibt.

In ähnlich kann beispielsweise auch die Emission der elektromagnetischen Strahlung der LEDs 35 beeinflusst werden, indem beispielsweise das in Figur 22 dargestellte Fenster für das Gehäuse 33 der Beleuchtungsvorrichtung 31 verwendet wird, wodurch es zu einer Ausleuchtung mit einem insgesamt größeren Öffnungswinkel kommt.

Alternativ kann der Halterungskörper 13 auch einen hochtemperaturbeständigen Kunststoff, insbesondere einen thermoplastischen Kunststoff wie einen Polyaryletherketon, insbesondere einen Polyetheretherketon, PEEK, umfassen oder aus diesem bestehen.

Wenn der Halterungskörper 13 einen hochtemperaturbeständigen Kunststoff, insbesondere einen thermoplastischen Kunststoff wie einen Polyaryletherketon, insbesondere einen Polyetheretherketon, PEEK, umfasst oder aus diesem besteht, muss dieser Halterungskörper 13 nicht unbedingt vollständig hermetisch sein, wie dieses im Rahmen der vorliegenden Offenbarung beschrieben wurde, sondern können dennoch durchaus wertvolle Betriebs- und Einsatzzeiten erreicht werden.

Es kann beispielsweise der PEEK umfassende oder aus PEEK bestehende Halterungskörper 13 eine vorzugsweise säulenförmige Durchgangsöffnung mit kreisförmigem Querschnitt aufweisen und der Außendurchmesser eines transparenten Elements 12 mit ebenfalls kreisförmigen seitlichen Außenabmessungen um etwa 1/10 größer sein als der Innendurchmesser der kreisförmigen Durchgangsöffnung des Halterungskörpers 13. Wenn der Halterungskörper 3 auf eine Temperatur von etwa 200°C erhitzt wird, kann dann das transparente Element 12 in diese Durchgangsöffnung eingesetzt werden und es entsteht bei Abkühlung eine wie vorstehend beschriebene Druckspannung, beispielsweise von etwa 38 MPa, welche noch deutlich unter der Streckgrenze von 110 MPa von PEEK liegt.

Bei den hier offenbarten Ausführungsformen kann die Beleuchtungsvorrichtung jeweils fest, insbesondere durch Verschweißen des Sockelteils 32 mit dem Halterungskörper 13 oder auch alternativ lösbar an dem Halterungskörper 13 gehalten sein. Zur lösbaren Befestigung der Beleuchtungsvorrichtung 31 am Halterungskörper 13 können Befestigungsmittel vorgesehen sein, die beispielsweise Passstifte umfassen, welche in zugeordnete Passnuten oder passgenaue Sackloch-Bohrungen des jeweils zweiten Gehäuses eingreifen können.

Beispielhaft ist in Figur 11 lediglich jeweils der Passstift 57, 58 des Halterungskörpers 13 dargestellt, welcher in eine zugeordnete jeweilige Bohrung 59, 60 des Sockelabschnitts 32 eingreift.

Die Erfindung betrifft generell eine Vorrichtung zur Halterung einer bilderfassenden Einrichtung an einem Bioreaktor, einen Bioreaktor mit einer Vorrichtung zur Halterung einer bilderfassenden Einrichtung sowie Verfahren zur Vermehrung oder Kultivierung biologischen Materials, wobei eine Vorrichtung zur Halterung einer bilderfassenden Einrichtung, insbesondere einer mikroskopischen Einrichtung, Verwendung findet, welche eine Halterung mit einem Fenster mit einem transparenten Element, das für elektromagnetische Strahlung transparent ist aufweist, wobei die Vorrichtung zur Halterung einer bilderfassenden Einrichtung zumindest teilweise in einer Durchgangsöffnung einer Durchführung eines Behälters zur Aufnahme von fluiden Medien, die biologisches Material enthalten, gehalten ist, wobei vorzugsweise das Fenster die Durchgangsöffnung verschließt.

Diese Anmeldung umfasst auch den Gegenstand der Anmeldungen gleichen Anmelderin mit dem Titel "Photobioreaktor mit Vorrichtung zur Abgabe elektromagnetischer Strahlung, Vorrichtung zur Abgabe elektromagnetischer Strahlung sowie Verfahren zur Vermehrung oder Kultivierung biologischen Materials, Verfahren zum Präparieren von biologischem Material und/oder Herstellen von Pharmazeutika, insbesondere Biopharmazeutika" und mit dem Titel "Sensoraufnahme für einen Bioreaktor sowie Bioreaktor mit Sensoraufnahme und Verfahren zur Vermehrung oder Kultivierung biologischen Materials" ,welche am selben Tag wie die vorliegende Anmeldung beim Deutschen Patent- und Markenamt eingereicht wurden und welche durch Inkorporierung mittels Bezugnahme in diese Anmeldung vollumfänglich aufgenommen werden.

### Bezugszeichenliste

- 1: Bioreaktor
- 2: Vorrichtung zur Halterung einer bilderfassenden Einrichtung
- 3: Behälter zur Aufnahme fluider Medien, die biologisches Material enthalten
- 4: das fluide Medium oder die fluiden Medien
- 5: biologisches Material
- 6: Port oder Durchführung
- 7: Durchgangsöffnung des Behälters 3
- 8: Mikroskopsonde
- 9: bilderfassenden Einrichtung, insbesondere einer mikroskopischen Einrichtung
- 10: Grundkörper des Fensters 11
- 11: Fenster
- 12: Transparentes Element
- 13: Halterungskörper
- 14: Durchgangsöffnung
- 15: Halterung
- 16: Reibelement, vorzugsweise O-Ring
- 17: Zylindrische Ausnehmung
- 18: Ringförmiges Druckelement
- 19: Sprengring
- 20: seitlicher, sich in radialer Richtung erstreckender Absatz
- 21: oberer Flansch
- 22: Hutmutter
- 23: Durchgangsöffnung
- 24: Gewinde
- 25: Gegengewinde
- 26: Sprengring
- 27: Dichtelement, insbesondere O-Ring
- 28: Innenwand des Bioreaktors 1
- 29: axialer Abstand des Fensters 11 zur Innenwand 28
- 30: axialer Abstand des Fensters 11 zum seitlichen Absatz 20
- 31: Beleuchtungsvorrichtung
- 32: Sockelabschnitt
- 33: Fußteil oder Gehäuse
- 34: Abstand
- 35: LED
- 36: Array von LEDs 35
- 37: Gedruckte Schaltungsplatine
- 38: Mehrfachleiter
- 39: Kontroller
- 40: Elektrischer Verbinder
- 41: Elektrischer Verbinder
- 42: Mehrfachleiter
- 43: Elastomere Komponente
- 44: Prozessteuerungseinrichtung(PST)
- 45: Weiterer Verbinder
- 46: Mehrfachleiter
- 47: Induktiver Koppler des Halterungskörpers 14
- 48: Induktiver Koppler des Gehäuses 33
- 49: Mehrfachleiter
- 50: Mehrfachleiter
- 51: Sensor zum Messen der Wellenlänge und/oder Intensität von elektromagnetischer Strahlung
- 52: Mehrfachleiter
- 53: Hauptoberfläche
- 54: Hauptoberfläche
- 55: Laserschweißnaht
- 56: Optisches Linsenelement
- 57: Passstift
- 58: Passstift
- 59: zugeordnete Passnuten oder passgenaue Sackloch-Bohrungen
- 60: zugeordnete Passnuten oder passgenaue Sackloch-Bohrungen
- 61: weiteres Glas oder Glaslot

- S: Symmetrieachse
- H: Hilfslinie

## Patentansprüche

1. Bioreaktor umfassend
einen Behälter zur Aufnahme von fluiden Medien, die biologisches Material enthalten,
eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten,
eine Vorrichtung zur Halterung einer bilderfassenden Einrichtung, insbesondere einer mikroskopischen Einrichtung, welche
ein Fenster mit einem transparenten Element, das für elektromagnetische Strahlung transparent ist sowie eine Halterung für die bilderfassende Einrichtung aufweist,
**dadurch gekennzeichnet, dass**
die Vorrichtung zur Halterung einer bilderfassenden Einrichtung zumindest teilweise in der Durchgangsöffnung der Durchführung gehalten ist, wobei das Fenster die Durchgangsöffnung verschließt.

2. Bioreaktor nach vorstehendem Anspruch, **dadurch gekennzeichnet, dass** der Bioreaktor zusammen mit der Vorrichtung zur Halterung einer bilderfassenden Einrichtung autoklavierbar ist, insbesondere während diese zumindest teilweise in der Durchgangsöffnung der Durchführung gehalten ist, autoklavierbar ist.

3. Vorrichtung zur Halterung einer bilderfassenden Einrichtung, insbesondere einer mikroskopischen Einrichtung, an einem Bioreaktor nach einem der vorstehenden Ansprüche,
bei welcher die Halterung für eine bilderfassende Einrichtung ein Fenster mit einem transparentem Element, das für elektromagnetische Strahlung transparent ist, umfasst und
die Vorrichtung zur Halterung einer bilderfassenden Einrichtung zumindest teilweise in der Durchgangsöffnung der Durchführung gehalten ist und das Fenster die Durchgangsöffnung verschließt.

4. Vorrichtung zur Halterung einer bilderfassenden Einrichtung nach Anspruch 3, **gekennzeichnet durch** einen Grundköper, an welchem das transparente Element des Fensters, hermetisch dicht angeordnet ist.

5. Vorrichtung zur Halterung einer bilderfassenden Einrichtung nach einem der beiden vorstehenden Ansprüche, **dadurch gekennzeichnet, dass** ein Halterungskörper der Vorrichtung zur Halterung einer bilderfassenden Einrichtung zylindersymmetrisch, insbesondere säulenförmig ausgebildet ist und eine Durchgangsöffnung aufweist, welche auf der dem Inneren des Bioreaktors zugeordneten Seite mittels des Fensters fluiddicht, insbesondere hermetisch dicht abgeschlossen ist.

6. Vorrichtung zur Halterung einer bilderfassenden Einrichtung nach Anspruch 3, 4 oder 5, **dadurch gekennzeichnet, dass** das transparente Element des Fensters in einem Spektralbereich mit einer Wellenlänge von 250 bis 2000 nm eine Transmission aufweist, die höher ist als 80%, besonders bevorzugt höher als 90 %.

7. Vorrichtung zur Halterung einer bilderfassenden Einrichtung nach einem der Ansprüche 4 bis 6, **dadurch gekennzeichnet, dass** das transparente Element des Fensters Glas umfasst oder aus Glas besteht und vorzugsweise Quarzglas oder Borosilikatglas umfasst oder aus diesem besteht.

8. Vorrichtung zur Halterung einer bilderfassenden Einrichtung nach einem der Ansprüche von 3 bis 7, **dadurch gekennzeichnet, dass** das transparente Element des Fensters scheibenförmig ausgebildet ist und insbesondere planparallele Hauptoberflächen aufweist oder
plankonvex, plankonkav, bikonvex, bikonkav, konvexkonkav oder konkavkonvex geformt ist.

9. Vorrichtung zur Halterung einer bilderfassenden Einrichtung nach einem der Ansprüche von 3 bis 8, **dadurch gekennzeichnet, dass** das transparente Element des Fensters Teil einer diesem zugeordneten mikroskopischen Einrichtung ist.

10. Vorrichtung zur Halterung einer bilderfassenden Einrichtung nach einem der Ansprüche von 3 bis 9, **gekennzeichnet durch** eine Beleuchtungsvorrichtung, welche an der dem Inneren des Behälters zur Aufnahme von fluiden Medien zugeordneten Seite am Halterungskörper angeordnet, vorzugsweise lösbar befestigbar angeordnet ist.

11. Vorrichtung zur Halterung einer bilderfassenden Einrichtung nach Anspruch 10, **dadurch gekennzeichnet, dass** die Beleuchtungsvorrichtung ein Gehäuse und ein weiteres Fenster, vorzugsweise mit einem weiteren Grundkörper und einem weiteren transparenten Element umfasst, welches dem Fenster der Vorrichtung zur Halterung einer bilderfassenden Einrichtung im montierten Zustand gegenüberliegt und vorzugsweise das transparente Element des Fensters, welches am Halterungskörper der Vorrichtung zur Halterung einer bilderfassenden Einrichtung angeordnet ist, mittels einer Druckverglasung, insbesondere GTMS-Druckverglasung am Grundkörper und dieser vorzugsweise durch eine Schweißverbindung am Halterungskörper gehalten ist und/oder
das transparente Element des Fensters der Beleuchtungsvorrichtung mittels einer Druckverglasung, insbesondere GTMS-Druckverglasung am Grundkörper und dieser vorzugsweise durch eine Schweißverbindung am Gehäuse der Beleuchtungsvorrichtung gehalten ist.

12. Vorrichtung zur Halterung einer bilderfassenden Einrichtung nach Anspruch 10 oder 11, **dadurch gekennzeichnet, dass** die Vorrichtung zur Halterung einer bilderfassenden Einrichtung und deren Beleuchtungsvorrichtung im montierten Zustand innerhalb des Behälters zur Aufnahme von fluiden Medien eine Kammer mit einem definierten, insbesondere zu zumindest zwei Seiten senkrecht zur Symmetrieachse des Halterungsköpers hin offenem Volumen ausbilden, wobei
vorzugsweise das transparente Element des Fensters der Beleuchtungsvorrichtung und das transparente Element des Fensters, welches am Halterungskörper der Vorrichtung zur Halterung einer bilderfassenden Einrichtung angeordnet ist, einen definierten Abstand zueinander aufweisen.

13. Vorrichtung zur Halterung einer bilderfassenden Einrichtung nach einem der Ansprüche von 10, 11 oder 12, **dadurch gekennzeichnet, dass** die Vorrichtung zur Halterung einer bilderfassenden Einrichtung an deren Halterungskörper zumindest einen elektrischen Verbinder und die Beleuchtungsvorrichtung an deren Gehäuse zumindest einen elektrischen Verbinder aufweisen, welche miteinander fügekompatibel und jeweils hermetisch abgedichtet sind und/oder die Vorrichtung zur Halterung einer bilderfassenden Einrichtung an deren Halterungskörper zumindest einen induktiven Koppler und die Beleuchtungsvorrichtung an deren Gehäuse zumindest einen induktiven Koppler aufweisen, welche miteinander im montierten Zustand induktiv koppelbar und jeweils hermetisch abgedichtet sind.

14. Vorrichtung zur Halterung einer bilderfassenden Einrichtung nach einem der Ansprüche von 10 bis 13, **dadurch gekennzeichnet, dass** die Beleuchtungsvorrichtung eine LED oder einen Array von LEDs umfasst, die in dem Gehäuse der Beleuchtungsvorrichtung angeordnet sind und
die LEDs vorzugsweise mittels einer zugeordneten elektronischen Einrichtung stroboskopisch ansteuerbar sind.

15. Verfahren zur Vermehrung oder Kultivierung biologischen Materials umfassend
das Einbringen von fluiden Medien, die biologisches Material oder eine Vorstufe biologischen Materials enthalten in einen Bioreaktor nach Anspruch 1 oder 2, insbesondere in einen Behälter des Bioreaktors zur Aufnahme von fluiden Medien, die biologisches Material enthalten,
wobei der Behälter zur Aufnahme von fluiden Medien, die biologisches Material enthalten,
eine Durchführung mit einer Durchgangsöffnung zwischen dem Inneren des Behälters zur Aufnahme von fluiden Medien, die biologisches Material enthalten, und dem Äußeren des Behälters zur Aufnahme von fluiden Medien, die biologisches Material enthalten aufweist, und
eine Vorrichtung zur Halterung einer bilderfassenden Einrichtung, nach einem der Ansprüche von 3 bis 14 umfasst, umfassend vorzugsweise die Herstellung von Pharmazeutika, insbesondere von Biopharmazeutika.

16. Verfahren nach Anspruch 15, bei welchem von einer Beleuchtungsvorrichtung, welche vorzugsweise an der dem Inneren des Behälters zur Aufnahme fluider Medien zugeordneten Seite am Halterungskörper angeordnet, vorzugsweise lösbar befestigbar angeordnet ist, elektromagnetische Strahlung abgegeben, vorzugsweise in Form von zeitlich beschränkten Pulsen abgegeben wird insbesondere in den Behälter zur Aufnahme fluider Medien eingestrahlt wird und vorzugsweise die zeitlich beschränkten Pulse der elektromagnetischen Strahlung in einer zeitsynchronen Weise und somit in festen Zeitintervallen oder auch in zeitlich asynchrone Weise und somit in variierenden Zeitintervallen abgegeben werden und
bei welchem das Erfassen von Bildinformationen mit der bilderfassenden Einrichtung, vorzugsweise während eines zeitlich beschränkten Intervalls synchron zur Abgabe der elektromagnetische Strahlung in Form von zeitlich beschränkten Pulsen erfolgt.

17. Verfahren nach Anspruch 15 oder 16, wobei die Vorrichtung zur Halterung einer bilderfassenden Einrichtung zumindest einen Sensor aufweist, und im Betriebszustand die Strahlungsintensität und/oder die Wellenlänge der elektromagnetischen Strahlung im Innern des Bioreaktors, insbesondere im Innern des Behälters zur Aufnahme fluider Medien, welche biologisches Material enthalten, gemessen, insbesondere ortsaufgelöst gemessen wird und bei welchem vorzugsweise für einen definierten Zeitraum elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird.

18. Verfahren nach einem der Ansprüche von 15 bis 17, bei welchem durch Mutagenese veränderte photo- oder mixotrophen Mikroorganismen insbesondere auch Mikroalgen, Hefen und Bakterien, verwendet werden.

## Claims

1. A bioreactor, comprising
a container for holding fluid media containing biological material;
a feedthrough with a through-opening between the interior of the container for holding fluid media containing biological material and the exterior of the container for holding fluid media containing biological material;
a device for mounting an image capturing device, in particular a microscopic device, comprising
a window including a transparent element that is transparent to electromagnetic radiation, and
a holder for the image capturing device;
**characterized in that**
the device for mounting an image capturing device is held at least partially in the through-opening of the feedthrough, whereby the window seals the through-opening.

2. The bioreactor according to the preceding claim, **characterized in that** the bioreactor is autoclavable together with the device for mounting an image capturing device, in particular autoclavable while the device for mounting an image capturing device is held at least partially in the through-opening of the feedthrough.

3. A device for mounting an image capturing device, in particular a microscopic device, in particular on a bioreactor according to any of the preceding claims, wherein the holder for an image capturing device comprises a window including a transparent element which is transparent to electromagnetic radiation; and
wherein the device for mounting an image capturing device is held at least partially in the through-opening of the feedthrough and the window seals the through-opening.

4. The device for mounting an image capturing device according to claim 3, **characterized by** a main body on which the transparent element of the window is secured in a hermetically sealed manner.

5. The device for mounting an image capturing device according to any one of the two preceding claims, **characterized in that** a holder body of the device for mounting an image capturing device has a cylindrically symmetrical shape, in particular a columnar shape, and has a through-opening which is sealed fluid-tightly, in particular hermetically, by the window on the side associated with the interior of the bioreactor.

6. The device for mounting an image capturing device according to claim 3, 4, or 5, **characterized in that** the transparent element of the window exhibits a transmittance of greater than 80 %, most preferably greater than 90 %, in a spectral range of wavelengths between 250 and 2000 nm.

7. The device for mounting an image capturing device according to any one of claims 4 to 6, **characterized in that** the transparent element of the window comprises glass or is made of glass, and preferably comprises fused silica or borosilicate glass.

8. The device for mounting an image capturing device according to any one of claims 3 to 7, **characterized in that** the transparent element of the window has a sheet-like shape and in particular plane-parallel main surfaces; or
is one of plano-convex, plano-concave, biconvex, biconcave, convexo-concave, or concavoconvex.

9. The device for mounting an image capturing device according to any one of claims 3 to 8, **characterized in that** the transparent element of the window forms part of a microscopic device associated therewith.

10. The device for mounting an image capturing device according to any one of claims 3 to 9, **characterized by** an illumination device which is disposed on the holder body, preferably in a detachably mountable manner, on the side associated with the interior of the container for holding fluid media.

11. The device for mounting an image capturing device according to claim 10, **characterized in that** the illumination device comprises a housing and a further window preferably comprising a further main body and a further transparent element, which in its installed state is arranged opposite the window of the device for mounting an image capturing device; and
that, preferably, the transparent element of the window disposed on the holder body of the device for mounting an image capturing device is secured to the main body by a compression glass seal, in particular a glass-to-metal seal, GTMS, compression glass seal, and the main body is secured to the holder body preferably by a welded joint; and/or
that the transparent element of the window of the illumination device is secured to the main body by a compression glass seal, in particular a GTMS compression glass seal, and the main body is secured to the housing of the illumination device preferably by a welded joint.

12. The device for mounting an image capturing device according to claim 10 or 11, **characterized in that** in their installed state within the container for holding fluid media, the device for mounting an image capturing device and the illumination device thereof define a chamber with a defined volume that is open in particular towards at least two sides perpendicular to the symmetry axis of the holder body; wherein,
preferably, the transparent element of the window of the illumination device and the transparent element of the window secured to the holder body of the device for mounting an image capturing device have a predefined spacing from one another.

13. The device for mounting an image capturing device according to any one of claims 10,11, or 12, **characterized in that** the device for mounting an image capturing device comprises at least one electrical connector on its holder body, and that the illumination device comprises at least one electrical connector on its housing, which electrical connectors are mateable with each other and are each hermetically sealed; and/or that
the device for mounting an image capturing device comprises at least one inductive coupler on its holder body and the illumination device comprises at least one inductive coupler on its housing, which inductive couplers can be coupled inductively and are each hermetically sealed in their mounted state.

14. The device for mounting an image capturing device according to any one of claims 10 to 13, **characterized in that** the illumination device comprises an LED or an array of LEDs arranged in the housing of the illumination device; and
that the LEDs are preferably controllable stroboscopically by an associated electronic device.

15. A method for propagation or cultivation of biological material, comprising introducing fluid media containing biological material or a precursor of biological material into a bioreactor according to claim 1 or 2, in particular into a container of the bioreactor for holding fluid media containing biological material;
wherein the container for holding fluid media containing biological material comprises a feedthrough with a through-opening between the interior of the container for holding fluid media containing biological material and the exterior of the container for holding fluid media containing biological material, and
a device for mounting an image capturing device according to any one of claims 3 to 14; preferably comprising the production of pharmaceuticals, in particular of biopharmaceuticals.

16. The method according to claim 15, wherein electromagnetic radiation is emitted by an illumination device which is preferably disposed on the holder body, preferably in a detachably mountable manner, on the side associated with the interior of the container for holding fluid media, in particular electromagnetic radiation is irradiated into the container for holding fluid media in the form of time-limited pulses; and wherein,
preferably, the time-limited pulses of the electromagnetic radiation are emitted in a time-synchronous manner and thus at fixed time intervals, or else in a time-asynchronous manner and thus at varying time intervals; and wherein
the capturing of image information using the image capturing device is preferably performed during a time-limited interval in synchronism with the emission of the electromagnetic radiation in the form of time-limited pulses.

17. The method according to claim 15 or 16, wherein the device for mounting an image capturing device comprises at least one sensor, and wherein, during operation, the radiation intensity and/or wavelength of the electromagnetic radiation is measured in the interior of the bioreactor, in particular in the container for holding fluid media, in particular measured in a spatially resolved manner; and wherein, preferably, electromagnetic radiation of a defined wavelength, preferably 250 nm, is irradiated into the bioreactor for a predefined period of time;
and after this irradiation, a radiation intensity and/or a wavelength of the electromagnetic radiation is measured within the interior of the bioreactor over a broad range of wavelengths or selectively at one wavelength, in particular at 270 nm.

18. The method according to any one of claims 15 to 17, wherein photo- or mixotrophic microorganisms modified by mutagenesis are used, in particular microalgae, yeasts and bacteria.

## Revendications

1. Bioréacteur, comprenant
un récipient destiné à recevoir des milieux fluides qui contiennent de la matière biologique,
une traversée dotée d'une ouverture de passage entre l'intérieur du récipient, destiné à recevoir des milieux fluides contenant de la matière biologique, et l'extérieur du récipient, destiné à recevoir des milieux fluides contenant de la matière biologique,
un dispositif de support d'un agencement d'acquisition d'image, en particulier d'un agencement microscopique qui présente une fenêtre, dotée d'un élément transparent qui est transparent aux rayonnement électromagnétique, ainsi qu'un support pour l'agencement d'acquisition d'image,
**caractérisé en ce que**
le dispositif de support d'un agencement d'acquisition d'image est retenu au moins en partie dans l'ouverture de passage de la traversée, la fenêtre fermant l'ouverture de passage.

2. Bioréacteur selon la revendication précédente, **caractérisé en ce que** le bioréacteur est autoclavable, conjointement avec le dispositif de support d'un agencement d'acquisition d'image, et est autoclavable en particulier pendant que celui-ci est retenu dans l'ouverture de passage de la traversée.

3. Dispositif de support d'un agencement d'acquisition d'image, en particulier d'un agencement microscopique, sur un bioréacteur selon l'une des revendications précédentes,
dans lequel le dispositif de support d'un agencement d'acquisition d'image comprend une fenêtre dotée d'un élément transparent qui est transparent aux rayonnement électromagnétique, et
le dispositif de support d'un agencement d'acquisition d'image est retenu au moins en partie dans l'ouverture de passage de la traversée, et la fenêtre ferme l'ouverture de passage.

4. Dispositif de support d'un agencement d'acquisition d'image selon la revendication 3, **caractérisé en ce qu'**il comprend un corps de base sur lequel l'élément transparent de la fenêtre est disposé de manière hermétique.

5. Dispositif de support d'un agencement d'acquisition d'image selon l'une des deux revendications précédentes, **caractérisé en ce qu'**un corps de support du dispositif de support d'un agencement d'acquisition d'image est réalisé avec une symétrie cylindrique, en particulier sous forme de colonne, et présente une ouverture de passage qui, sur le côté associé à l'intérieur du bioréacteur, est fermée de manière étanche aux fluides, notamment de manière hermétique, au moyen de la fenêtre.

6. Dispositif de support d'un agencement d'acquisition d'image selon la revendication 3, 4 ou 5, **caractérisé en ce que** l'élément transparent de la fenêtre présente, dans un domaine spectral avec une longueur d'onde allant de 250 à 2 000 nm, une transmission qui est supérieure à 80 %, et de manière particulièrement avantageuse supérieure à 90 %.

7. Dispositif de support d'un agencement d'acquisition d'image selon l'une des revendications 4 à 6, **caractérisé en ce que** l'élément transparent de la fenêtre comprend du verre ou est constitué de verre et comprend de préférence du verre de quartz ou du verre borosilicaté ou est constitué de celui-ci.

8. Dispositif de support d'un agencement d'acquisition d'image selon l'une des revendications 3 à 7, **caractérisé en ce que** l'élément transparent de la fenêtre est réalisé sous forme de disque et présente notamment des surfaces principales planes parallèles ou est réalisé sous une forme plane convexe, plane concave, biconvexe, biconcave, convexe-concave ou concave-convexe.

9. Dispositif de support d'un agencement d'acquisition d'image selon l'une des revendications 3 à 8, **caractérisé en ce que** l'élément transparent de la fenêtre fait partie d'un agencement microscopique associé à celle-ci.

10. Dispositif de support d'un agencement d'acquisition d'image selon l'une des revendications 3 à 9, **caractérisé en ce qu'**il comprend un moyen d'éclairage qui est disposé sur le côté du corps de support associé à l'intérieur du récipient destiné à recevoir des milieux fluides, de préférence en pouvant être fixé de manière amovible.

11. Dispositif de support d'un agencement d'acquisition d'image selon la revendication 10, **caractérisé en ce que** le moyen d'éclairage comprend un boîtier et une autre fenêtre qui comporte de préférence un autre corps de base et un autre élément transparent et qui, à l'état monté, est située en vis-à-vis de la fenêtre du dispositif de support d'un agencement d'acquisition d'image, et l'élément transparent de la fenêtre, qui est disposée sur le corps de support du dispositif de support d'un agencement d'acquisition d'image, est maintenu à l'aide d'un verre comprimé, notamment d'un verre comprimé GTMS, sur le corps de base et celui-ci est maintenu de préférence par un assemblage soudé sur le corps de support, et/ou l'élément transparent de la fenêtre du moyen d'éclairage est maintenu à l'aide d'un verre comprimé, notamment d'un verre comprimé GTMS, sur le corps de base et celui-ci est maintenu de préférence par un assemblage soudé sur le boîtier du moyen d'éclairage.

12. Dispositif de support d'un agencement d'acquisition d'image selon la revendication 10 ou 11, **caractérisé en ce que**, à l'état monté, le dispositif de support d'un agencement d'acquisition d'image et son moyen d'éclairage forment à l'intérieur du récipient destiné à recevoir des milieux fluides, une chambre ayant un volume défini qui est ouvert notamment vers au moins deux côtés, perpendiculairement à l'axe de symétrie du corps de support, sachant que
l'élément transparent de la fenêtre du moyen d'éclairage et l'élément transparent de la fenêtre disposée sur le corps de support du dispositif de support d'un agencement d'acquisition d'image présentent une distance définie l'un par rapport à l'autre.

13. Dispositif de support d'un agencement d'acquisition d'image selon l'une des revendications 10, 11 ou 12, **caractérisé en ce que** le dispositif de support d'un agencement d'acquisition d'image présente au moins un connecteur électrique sur son corps de support, et le moyen d'éclairage présente au moins un connecteur électrique sur son boîtier, lesquels connecteurs sont compatibles avec un assemblage réciproque et sont chacun rendus hermétiques et/ou le dispositif de support d'un agencement d'acquisition d'image présente au moins un coupleur inductif sur son corps de support, et le moyen d'éclairage présente au moins un corps inductif sur son boîtier, lesquels coupleurs peuvent être couplés de manière inductive l'un à l'autre à l'état monté et sont chacun rendus hermétiques.

14. Dispositif de support d'un agencement d'acquisition d'image selon l'une des revendications 10 à 13, **caractérisé en ce que** le moyen d'éclairage comprend une LED ou un réseau de LEDs qui sont disposées dans le boîtier du moyen d'éclairage, et les LEDs peuvent de préférence être activées par voie stroboscopique à l'aide d'un dispositif électronique associé.

15. Procédé de multiplication ou de culture de matière biologique, comprenant
l'introduction de milieux fluides, contenant de la matière biologique ou un précurseur de matière biologique, dans un bioréacteur selon la revendication 1 ou 2, en particulier dans un récipient du bioréacteur destiné à recevoir des milieux fluides qui contiennent de la matière biologique,
selon lequel le récipient, qui est destiné à recevoir des milieux fluides contenant de la matière biologique, présente une traversée dotée d'une ouverture de passage entre l'intérieur du récipient, destiné à recevoir des milieux fluides contenant de la matière biologique, et l'extérieur du récipient, destiné à recevoir des milieux fluides contenant de la matière biologique, et
comporte un dispositif de support d'un agencement d'acquisition d'image selon l'une des revendications 3 à 14, comprenant de préférence la fabrication de produits pharmaceutiques, notamment de produits biopharmaceutiques.

16. Procédé selon la revendication 15, **caractérisé en ce qu'**un moyen d'éclairage, qui est de préférence disposé sur le côté du corps de support associé à l'intérieur du récipient destiné à recevoir des milieux fluides, de préférence en pouvant être fixé de manière amovible, émet un rayonnement électromagnétique, et l'émet de préférence sous forme d'impulsions limitées dans le temps, et l'envoie notamment dans le récipient destiné à recevoir des milieux fluides, et que les impulsions limitées dans le temps du rayonnement électromagnétique sont de préférence émises de manière synchrone, c'est-à-dire à intervalles de temps fixes, ou bien également de manière asynchrone, c'est-à-dire à des intervalles de temps variables, et selon lequel l'acquisition d'informations d'image avec l'agencement d'acquisition d'images a lieu de préférence pendant un intervalle limité dans le temps, de façon synchrone avec l'émission du rayonnement électromagnétique, sous forme d'impulsions limitées dans le temps.

17. Procédé selon la revendication 15 ou 16, selon lequel le dispositif de support d'un agencement d'acquisition d'image présente au moins un capteur et qu'en mode service on mesure, notamment avec une résolution spatiale, l'intensité de rayonnement et/ou la longueur d'onde du rayonnement électromagnétique à l'intérieur du bioréacteur, notamment à l'intérieur du récipient destiné à recevoir des milieux fluides contenant de la matière biologique, et selon lequel un rayonnement électromagnétique d'une longueur d'onde définie, de préférence de 250 nm, est envoyé dans le bioréacteur, de préférence pendant un laps de temps défini, et, après cette application de rayonnement, on mesure dans une large bande ou de manière sélective avec une longueur d'onde, notamment de 270 nm, une intensité de rayonnement et/ou une longueur d'onde du rayonnement électromagnétique à l'intérieur du bioréacteur.

18. Procédé selon l'une des revendications 15 à 17, selon lequel on utilise des micro-organismes phototrophes ou mixotrophes, modifiés par mutagénèse, en particulier également des micro-algues, des levures et des bactéries.
